# EUROPEAN PATENT SPECIFICATION

(11) **EP 1 254 903 B1**
(45) Date of publication and mention of the grant of the patent: **08.06.2005**
(21) Application number: 02405291.2
(22) Date of filing: 11.04.2002
(51) Int. Cl.: C07D 401/04, A61K 31/44, A61K 31/505

(54) **4-Amino-2-(pyridin-2-yl)pyrimidine as microbicidal active substances**
4-Amino-2-(2-pyridinyl)pyrimidine als mikrobizide Wirksubstanzen
4-Amino-2-(pyridin-2-yl)pyrimidine comme agents antibactérielles

(30) Priority: 20.04.2001 EP 01810387
(43) Date of publication of application: 06.11.2002
(73) Proprietor: Ciba Specialty Chemicals Holding Inc., 4057 Basel (CH)
(72) Inventor: Haap, Wolfgang, 79639 Grenzach-Wyhlen (DE); Hölzl, Werner, 68440 Eschentzwiller (FR); Petzold, Karin, 79592 Fischingen (DE)

(56) References cited:
- EP-A- 0 588 146
- DE-A- 4 029 649
- US-A- 5 250 530
- US-A- 5 346 899

## Description

The present invention relates to substituted 4-amino-2-(2-pyridyl)pyrimidines, to the preparation of such compounds, and to their use for the antimicrobial treatment of surfaces, as antimicrobial active substances against gram-positive and gram-negative bacteria, yeasts and fungi and also in the preservation of cosmetics, household products, textiles and plastics and for use in disinfectants.

DE 40 29 649 discloses alkxoxypyridyl-pyrimidine derivatives. The compounds are useful as fungicides.

US 5,250,530 discloses specific aminopyrimidine derivatives, their preparation, and agents containing them. The compounds are useful as fungicides.

EP 0 588 146 discloses substituted pyridine derivatives and pesticides containing them.

None of these references discloses 2-pyridylpyrimidines, wherein
a) the pyridyl-moiety is unsubstituted and simultaneously
b) has aminosubstituents (NR₃R₄) as the present claimed compounds.

The substituted 4-amino-2-(2-pyridyl)pyrimidines according to the invention correspond to formula 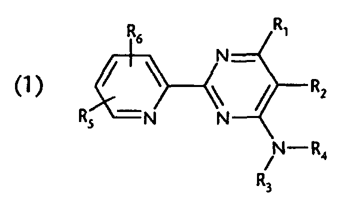 wherein
- R₁ and R₂: are each independently of the other hydrogen; unsubstituted or mono- or poly-halo-substituted C₁-C₂₀alkyl, C₁-C₂₀alkoxy, C₂-C₂₀alkenyl, C₂-C₂₀alkynyl, C₃-C₁₈cycloalkyl, C₃-C₇cycloalkyl-C₁-C₂₀alkyl; hydroxy; C₁-C₆alkoxy-C₁-C₂₀alkyl; carboxy; C₁-C₆alkyloxycarbonyl; cyano; mono- or di-C₁-C₂₀alkylamino; C₁-C₆alkylamino-C₁-C₂₀alkyl; halogen; phenyl; unsubstituted or C₁-C₅alkyl-, halo- or hydroxy-substituted phenyl-C₁-C₂₀alkyl, phenoxy or phenyl-C₁-C₂₀alkoxy; or R₁ and R₂ form a polymethylene chain of formula -(CH₂)ₘ- wherein m = 2-12;
- R₃: is unsubstituted C₇-C₂₀alkyl; or amino-, hydroxy-, carboxy- or C₁-C₆alkyloxycarbonyl-substituted C₂-C₂₀alkyl, C₈-C₁₈cycloalkyl, C₈-C₂₀alkenyl, C₈-C₂₀alkynyl, C₃-C₇cycloalkyl-C₈-C₂₀alkyl, C₁-C₄alkoxy-C₈-C₂₀alkyl, R₇R₈N-C₇-C₂₀alkyl, phenyl, phenyl-C₁-C₄alkyl or phenyl-C₁-C₄alkoxy;
- R₄: is hydrogen; unsubstituted or C₁-C₅alkyl-, halo- or hydroxy-substituted C₁-C₂₀alkyl, C₂-C₂₀alkenyl, C₂-C₂₀alkynyl, C₃-C₂₀cycloalkyl, C₃-C₇cycloalkyl-C₁-C₂₀alkyl, C₁-C₂₀alkoxy-C₁-C₆alkyl or R₇R₈N-C₁-C₂₀alkyl, phenyl, phenyl-C₁-C₂₀alkyl or phenoxy-C₁-C₂₀alkyl;
- R₅ and R₆: are each independently of the other hydrogen; and
- **R**_{**7**} **and R**_{**8**}: **are each independently of the other hydrogen; C1-C**_{**20**}**alkyl; C**_{**3**}**-C**_{**20**}**alkenyl; C**_{**3**}**- C**_{**20**}**alkynyl; C**_{**3**}**-C**_{**7**}**cycloalkyl; C**_{**3**}**-C**_{**20**}**cycloalkyl-C**_{**1**}**-C**_{**4**}**alkyl; phenyl; or phenyl-C**_{**1**}**-C**_{**4**}**alkyl.**

C₁-C₂₀Alkyl radicals are straight-chain or branched alkyl radicals, for example methyl, ethyl, n-propyl, isopropyl, n-butyl, sec-butyl, tert-butyl, amyl, isoamyl or tert-amyl, heptyl, octyl, isooctyl, nonyl, decyl, undecyl, dodecyl, tetradecyl, pentadecyl, hexadecyl, heptadecyl, octadecyl or eicosyl.

C₃-C₁₈Cycloalkyl denotes, for example, cyclopropyl, cyclobutyl, cyclopentyl, cycloheptyl, cyclooctyl, cyclononyl, cyclodecyl, cycloundecyl, cyclododecyl, cyclotetradecyl, cyclopentadecyl, cyclohexadecyl, cycloheptadecyl, cyclooctadecyl or, especially, cyclohexyl.

Alkenyl includes, within the scope of the meanings given, *inter alia,* allyl, isopropenyl, 2-butenyl, 3-butenyl, isobutenyl, n-penta-2,4-dienyl, 3-methyl-but-2-enyl, n-oct-2-enyl, n-dodec-2-enyl, isododecenyl, n-dodec-2-enyl and n-octadec-4-enyl.

C₁-C₅Alkoxy radicals are straight-chain or branched radicals, for example methoxy, ethoxy, propoxy, butoxy or pentyloxy.

Alkynyl includes, for example, ethynyl, propargyl, 2-butynyl, 1-pentynyl and 2-pentynyl.
- R₁ and R₂: in formula (1) are, each independently of the other, preferably hydrogen, unsubstituted or mono- or poly-halo-substituted C₁-C₂₀alkyl; or C₁-C₅alkoxy-C₁-C₅alkyl; especially hydrogen; unsubstituted or mono- or poly-halo-substituted C₁-C₅alkyl; or C₁-C₃alkoxy-C₁-C₅alkyl; and more especially hydrogen, methyl, ethyl, isopropyl, tert-butyl, CF₃ or the radical -(CH₂)ₘ-O-CH₃ wherein m is from 1 to 4.
- R₃: in formula (1) is preferably unsubstituted or amino-substituted C₇-C₂₀alkyl, C₃-C₇cycloalkyl-C₈-C₂₀alkyl, C₁-C₄alkoxy-C₇-C₂₀alkyl, R₇R₈N-C₇-C₂₀alkyl, phenyl-C₁-C₄alkyl or phenyl-C₁-C₆alkoxy, especially unsubstituted or amino-substituted C₇-C₂₀alkyl or R₇R₈N-C₇-C₂₀alkyl.

Especially preferred compounds of formula (1) are those wherein
- R₁ and R₂: are each independently of the other hydrogen, methyl, ethyl, isopropyl, tert-butyl, CF₃ or the radical -(CH₂)ₘ-O-CH₃;
- R₃: is unsubstituted or amino-substituted C₇-C₂₀alkyl or R₇R₈N-C₇-C₂₀alkyl;
- R₄: is hydrogen or C₁-C₂₀alkyl;
- **R**_{**5**} **and R**_{**6**}: are hydrogen;
- **R**_{**7**} **and R**_{**8**}: **are each independently of the others hydrogen or C**_{**1**}**-C**_{**20**}**alkyl;** and
- m: is from 1 to 4.

Special preference is given to compounds of formulae 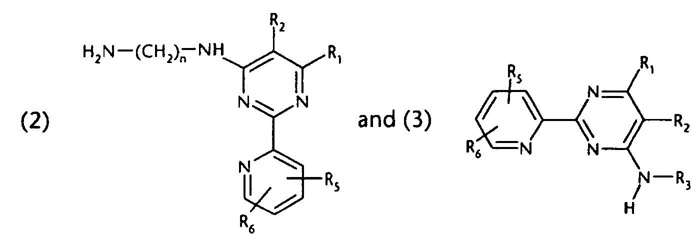 wherein
- n: is from 7 to 20; and
- R₁, R₂, R₃, R₅ and R₆: are as defined for formula (1).

All those compounds may also be present in the form of their acid addition salts, suitable acids being: HF, HCl, HBr, H₂SO₄, H₃PO₄, mono- and di-functional carboxylic acids, for example lactic acid, tartaric acid, acetic acid, maleic acid, fumaric acid, citric acid and salicylic acid, or sulfonic acid.

Table 1 below lists further 4-amino-2-(2-pyridyl)pyrimidines according to the invention by way of example:

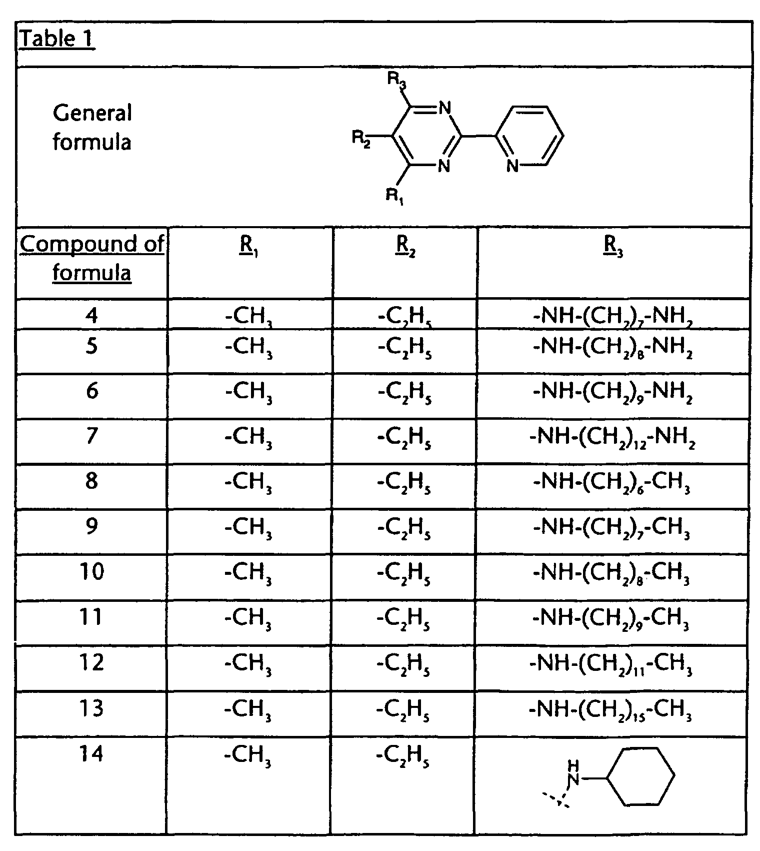

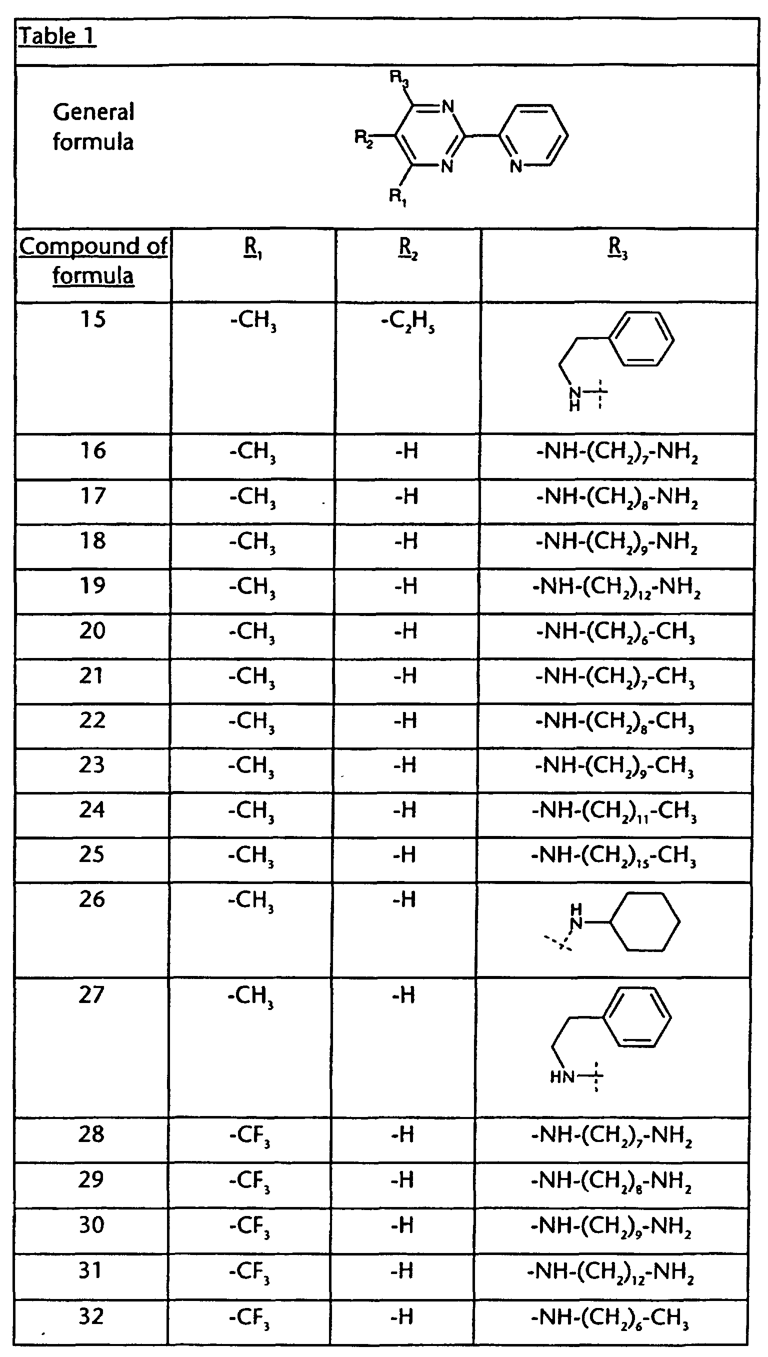

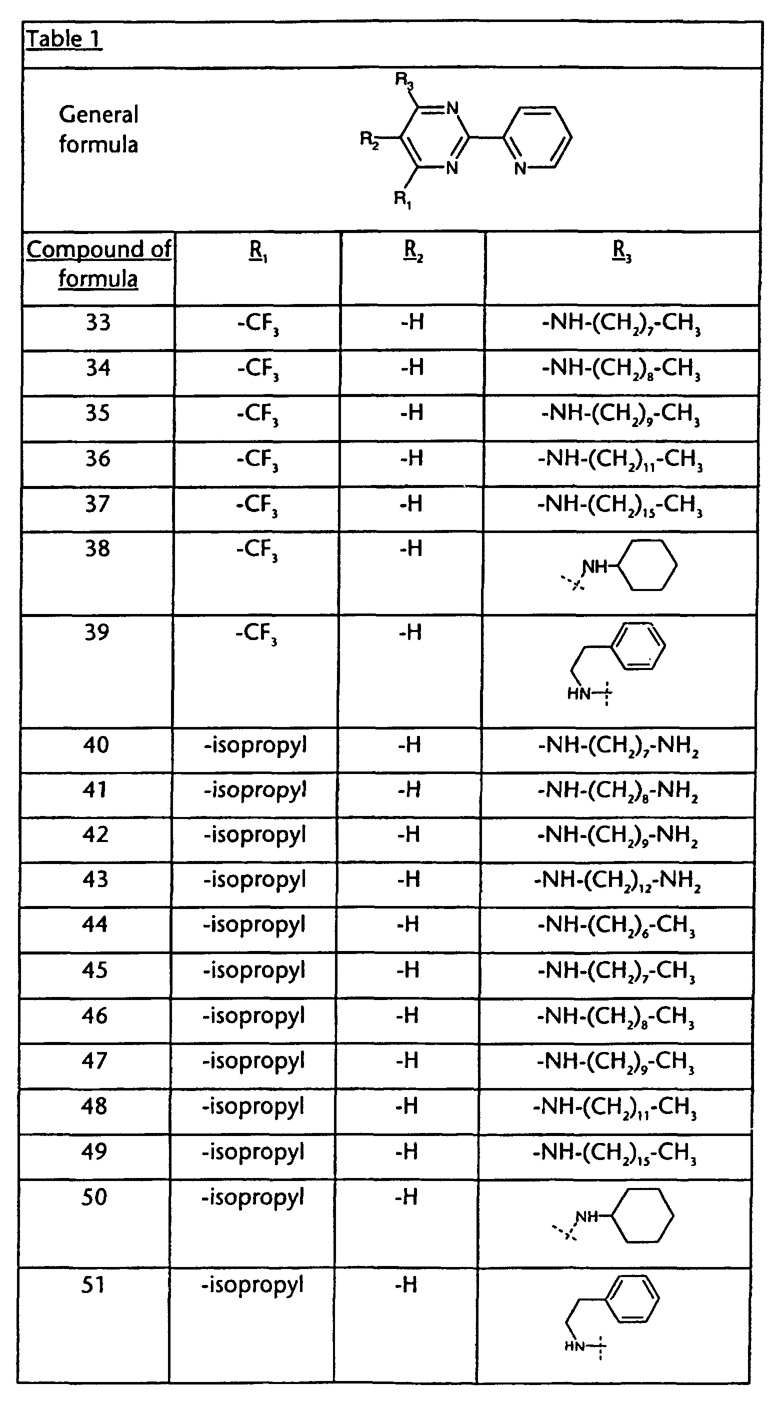

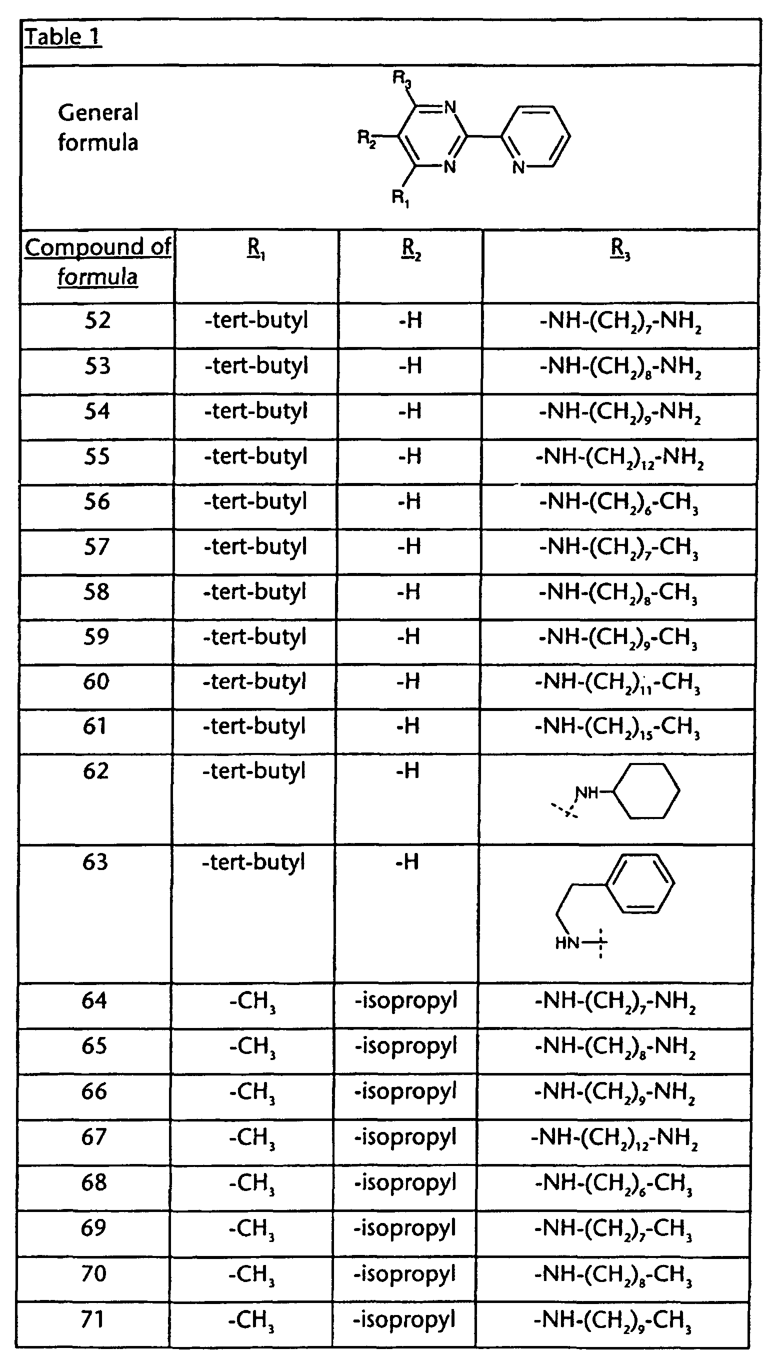

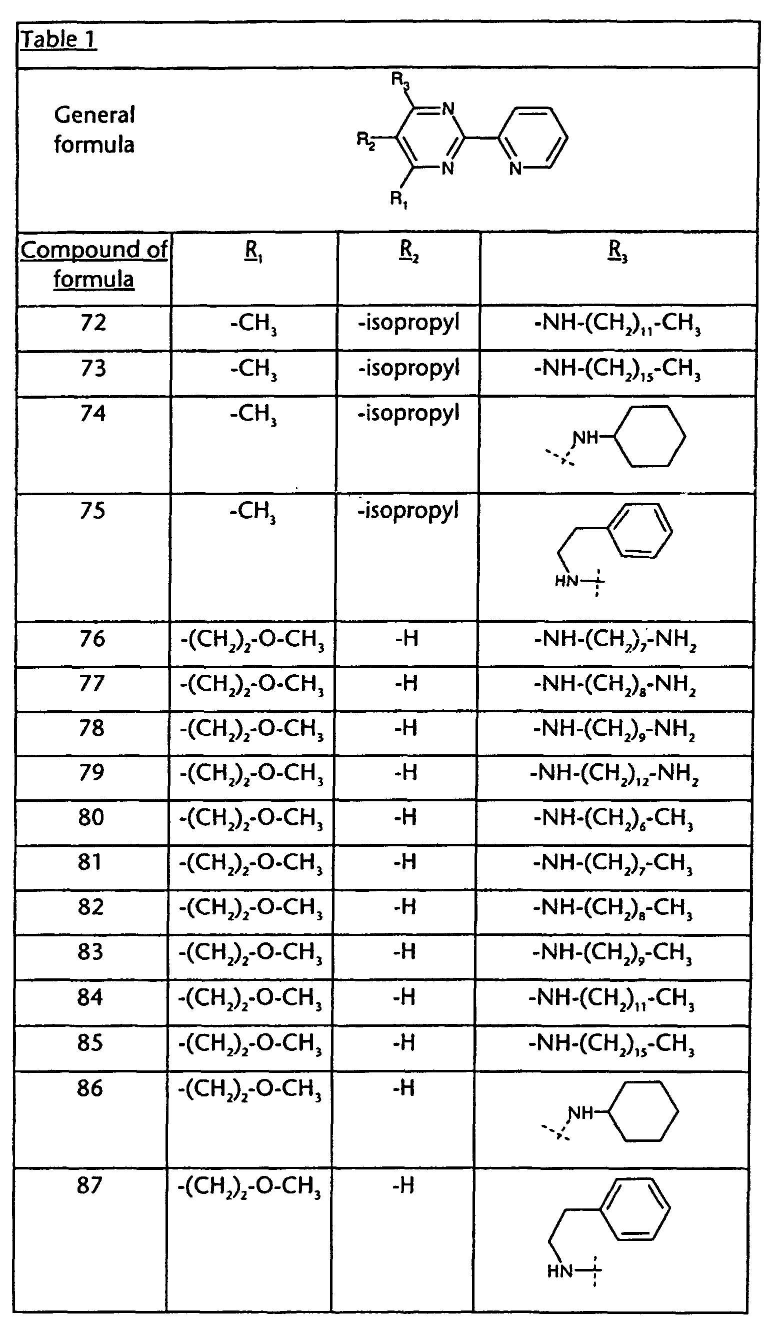

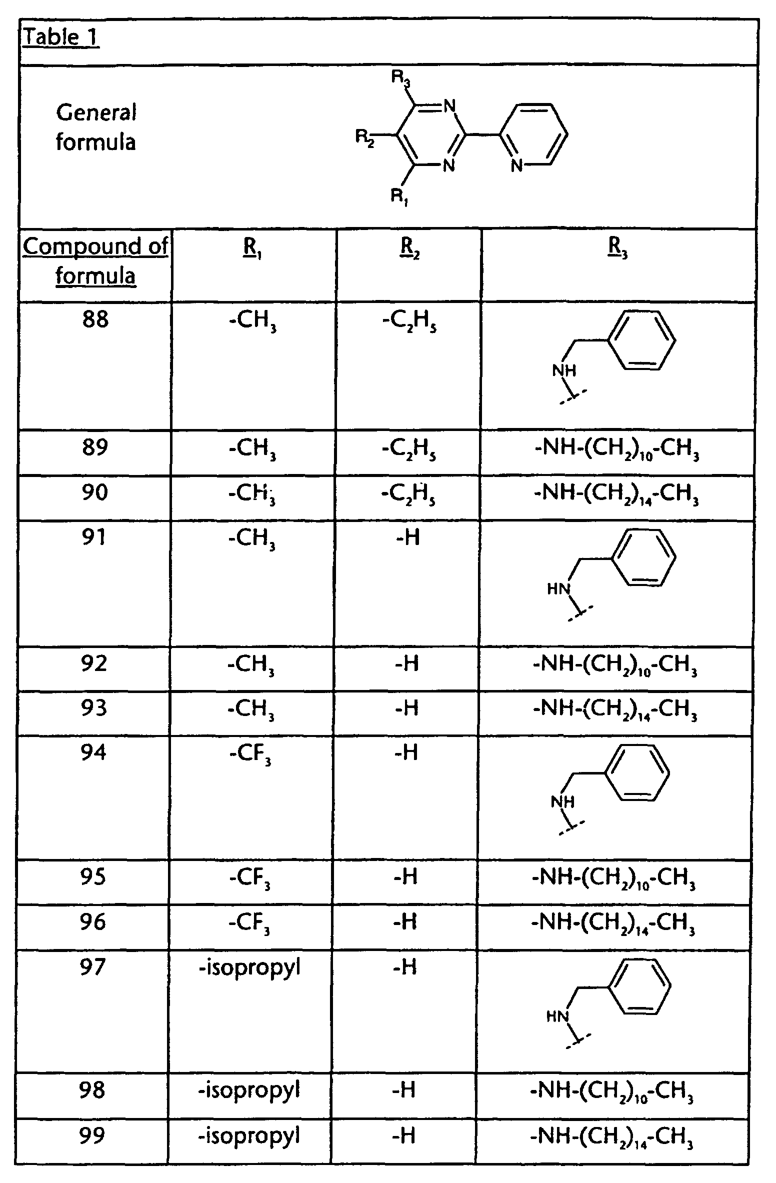

The novel 4-amino-2-(2-pyridyl)pyrimidines are prepared by methods known *per se* (J. Org. Chem.; 1967, 32, 1591). For that purpose, 2-cyanopyridine is reacted, in a suitable solvent, for example methanol, ethanol, isopropanol, DMF, tetrahydrofuran etc., with ammonium acetate or ammonium chloride at a temperature of from -10°C to 100°C over a period of from 1 hour to 24 hours to form the corresponding 2-amidinopyridine. The 2-amidinopyridine is then condensed with an appropriate β-keto ester using an auxiliary base, for example sodium carbonate, potassium hydroxide, sodium ethanolate, sodium methanolate, potassium tert-butanolate etc., in a suitable solvent, for example methanol, ethanol, butanol, tert-butanol, THF, DMF, acetonitrile, toluene, xylene etc., over a period of from 1 to 24 hours at a temperature of from 40 to 120°C. The 4-hydroxy-2-(2-pyridyl)pyrimidine thereby obtained is then converted into the corresponding 4-chloro-2-(2-pyridyl)pyrimidine by conventional methods using phosphorus oxychloride. The substituted 4-amino-2-(2-pyridyl)pyrimidines are obtained by reacting the 4-chloro-2-(2-pyridyl)pyrimidine with primary or secondary amines in a suitable solvent, for example DMF, dioxane, toluene, xylene, ethanol, butanol, and an auxiliary base, for example triethylamine, DIEA, sodium carbonate, potassium hydroxide etc., or using an excess of amine at from 40 to 130°C over a period of from 1 to 24 hours. Preparation of the compounds of formula (2), except for the reaction with polymer-bound diamines, is analogous to that of compound (1). The polymer-bound diamines are obtained by reacting an excess of from 2 to 10 equivalents of diamine in, for example, DMF, dichloromethane, THF or dioxane with trityl chloride polystyrene resin at a temperature of from 10 to 50°C over a period of from 0.5 to 24 hours. From 2 to 10 equivalents of appropriately substituted 4-chloro-2-(2-pyridyl)pyrimidines are then reacted, in a suitable solvent, for example dichloromethane, DMF, THF or toluene, with the polymer-bound diamines at from 10 to 120°C over a period of from 2 to 48 hours. After washing the resin to remove the excess, the target compounds are split off using from 1 to 30 % trifluoroacetic acid in dichloromethane at 25°C over a period of from 1 to 5 hours. For the purpose of further purification, the substances are freeze-dried from *t*BuOH/water 4:1 with from 1 to 10 % HOAc and once from *t*BuOH/water 4:1.

The entire reaction proceeds according to the following scheme :

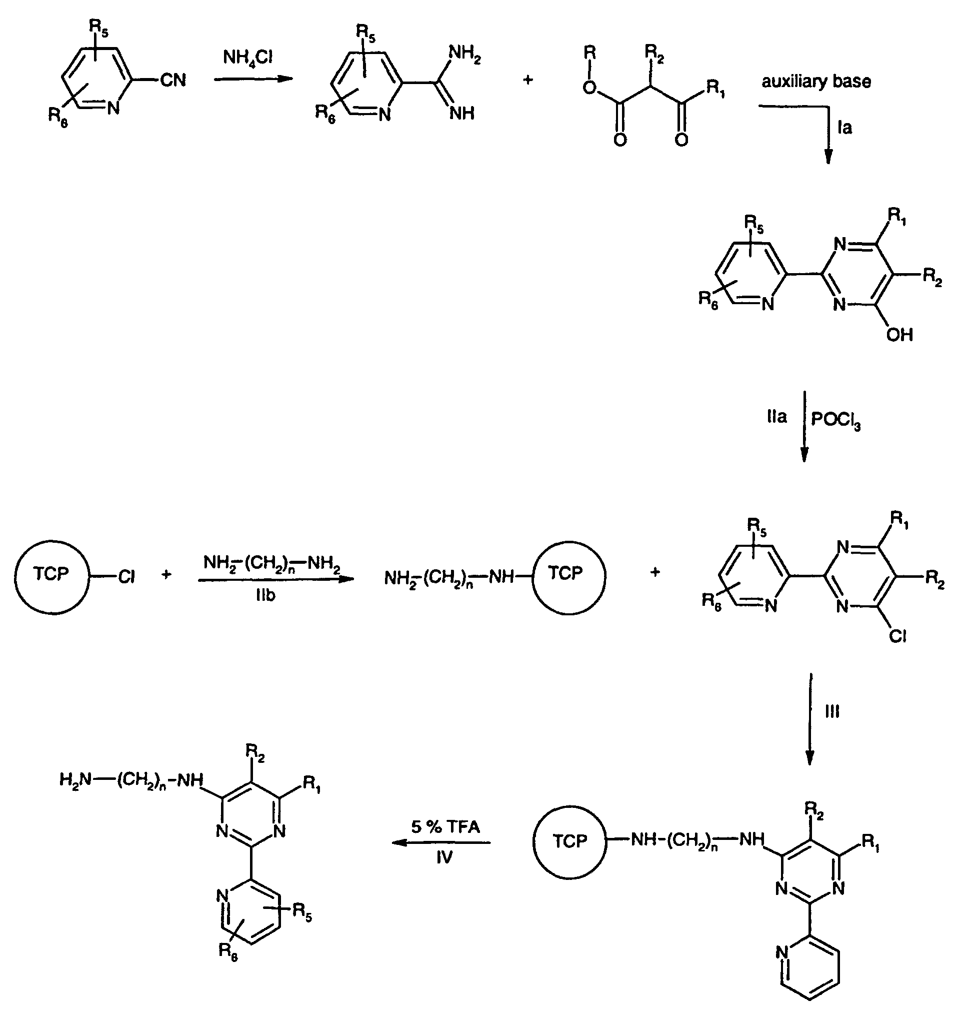

The compounds of formula (3) are prepared analogously to preparation of the compounds of formula (1) according to the following scheme:

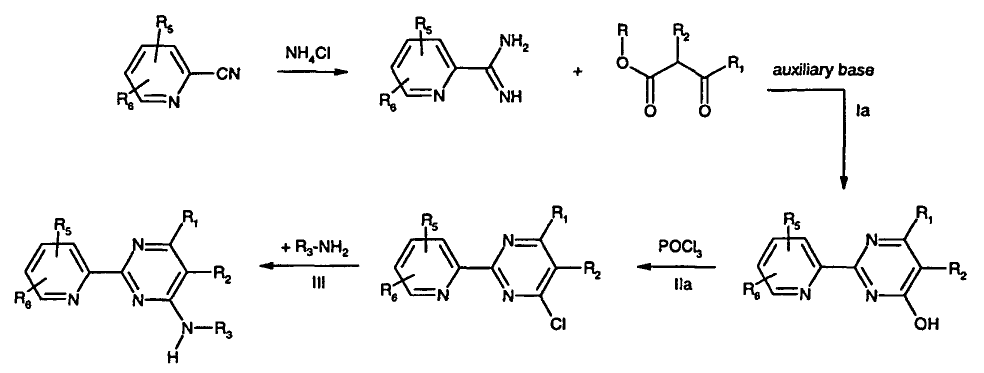

The 4-amino-2-(2-pyridyl)pyrimidines used in accordance with the invention exhibit pronounced antimicrobial action, especially against pathogenic gram-positive and gram-negative bacteria and against bacteria of the skin flora, and also against yeasts and moulds. They are accordingly suitable especially for disinfection, deodorisation, and for general and antimicrobial treatment of the skin and mucosa and of integumentary appendages (hair), more especially for the disinfection of hands and wounds.

They are accordingly suitable as antimicrobial active substances and preservatives in personal care preparations, for example shampoos, bath additives, haircare preparations, liquid and solid soaps (based on synthetic surfactants and salts of saturated and/or unsaturated fatty acids), lotions and creams, deodorants, other aqueous or alcoholic solutions, e.g. cleansing solutions for the skin, moist cleaning cloths, oils or powders.

The invention accordingly relates also to a personal care preparation comprising at least one compound of formula (1) and cosmetically tolerable carriers or adjuvants.

The personal care preparation according to the invention contains from 0.01 to 15 % by weight, preferably from 0.1 to 10 % by weight, based on the total weight of the composition, of a compound of formula (1), and cosmetically tolerable adjuvants.

Depending upon the form of the personal care preparation, it comprises, in addition to the 4-amino-2-(2-pyridyl)pyrimidine of formula (1), further constituents, for example sequestering agents, colourings, perfume oils, thickening or solidifying agents (consistency regulators), emollients, UV-absorbers, skin protective agents, antioxidants, additives that improve the mechanical properties, such as dicarboxylic acids and/or aluminium, zinc, calcium or magnesium salts of C₁₄-C₂₂fatty acids, and, optionally, preservatives.

The personal care preparation according to the invention may be in the form of a water-in-oil or oil-in-water emulsion, an alcoholic or alcohol-containing formulation, a vesicular dispersion of an ionic or non-ionic amphiphilic lipid, a gel, a solid stick or an aerosol formulation.

As a water-in-oil or oil-in-water emulsion, the cosmetirally tolerable adjuvant contains preferably from 5 to 50 % of an oil phase, from 5 to 20 % of an emulsifier and from 30 to 90 % water. The oil phase may comprise any oil suitable for cosmetic formulations, for example one or more hydrocarbon oils, a wax, a natural oil, a silicone oil, a fatty acid ester or a fatty alcohol. Preferred mono- or poly-ols are ethanol, isopropanol, propylene glycol, hexylene glycol, glycerol and sorbitol.

Cosmetic formulations according to the invention are used in various fields. There come into consideration, for example, especially the following preparations:
- skin-care preparations, e.g. skin-washing and cleansing preparations in the form of tablet-form or liquid soaps, synthetic detergents or washing pastes,
- bath preparations, e.g. liquid (foam baths, milks, shower preparations) or solid bath preparations, e.g. bath cubes and bath salts;
- skin-care preparations, e.g. skin emulsions, multi-emulsions or skin oils;
- cosmetic personal care preparations, e.g. facial make-up in the form of day creams or powder creams, face powder (loose or pressed), rouge or cream make-up, eye-care preparations, e.g. eyeshadow preparations, mascaras, eyeliners, eye creams or eye-fix creams; lip-care preparations, e.g. lipsticks, lip gloss, lip contour pencils, nail-care preparations, such as nail varnish, nail varnish removers, nail hardeners or cuticle removers;
- intimate hygiene preparations, e.g. intimate washing lotions or intimate sprays;
- foot-care preparations, e.g. foot baths, foot powders, foot creams or foot balsams, special deodorants and antiperspirants or callus-removing preparations;
- light-protective preparations, such as sun milks, lotions, creams or oils, sun-blocks or tropicals, pre-tanning preparations or after-sun preparations;
- skin-tanning preparations, e.g. self-tanning creams;
- depigmenting preparations, e.g. preparations for bleaching the skin or skin-lightening preparations;
- insect-repellents, e.g. insect-repellent oils, lotions, sprays or sticks;
- deodorants, such as deodorant sprays, pump-action sprays, deodorant gels, sticks or roll-ons;
- antiperspirants, e.g. antiperspirant sticks, creams or roll-ons;
- preparations for cleansing and caring for blemished skin, e.g. synthetic detergents (solid or liquid), peeling or scrub preparations or peeling masks;
- hair-removal preparations in chemical form (depilation), e.g. hair-removing powders, liquid hair-removing preparations, cream- or paste-form hair-removing preparations, hair-removing preparations in gel form or aerosol foams;
- shaving preparations, e.g. shaving soap, foaming shaving creams, non-foaming shaving creams, foams and gels, preshave preparations for dry shaving, aftershaves or aftershave lotions;
- fragrance preparations, e.g. fragrances (eau de Cologne, eau de toilette, eau de parfum, parfum de toilette, perfume), perfume oils or perfume creams;
- dental care, denture-care and mouth-care preparations, e.g. toothpastes, gel toothpastes, tooth powders, mouthwash concentrates, anti-plaque mouthwashes, denture cleaners or denture fixatives;
- cosmetic hair-treatment preparations, e.g. hair-washing preparations in the form of shampoos and conditioners, hair-care preparations, e.g. pretreatment preparations, hair tonics, styling creams, styling gels, pomades, hair rinses, treatment packs, intensive hair treatments, hair-structuring preparations, e.g. hair-waving preparations for permanent waves (hot wave, mild wave, cold wave), hair-straightening preparations, liquid hair-setting preparations, hair foams, hairsprays, bleaching preparations, e.g. hydrogen peroxide solutions, lightening shampoos, bleaching creams, bleaching powders, bleaching pastes or oils, temporary, semi-permanent or permanent hair colorants, preparations containing self-oxidising dyes, or natural hair colorants, such as henna or camomile.

An antimicrobial soap has, for example, the following composition:
0.01 to 5 % by weight of a compound of formula (1)
0.3 to 1 % by weight titanium dioxide,
1 to 10 % by weight stearic acid,
soap base ad 100 %, e.g. a sodium salt of tallow fatty acid or coconut fatty acid, or glycerol.

A shampoo has, for example, the following composition:
0.01 to 5 % by weight of a compound of formula (1),
12.0 % by weight sodium laureth-2-sulfate,
4.0 % by weight cocamidopropyl betaine,
3.0 % by weight NaCl and
water ad 100 %.

A deodorant has, for example, the following composition:
0.01 to 5 % by weight of a compound of formula (1),
60 % by weight ethanol,
0.3 % by weight perfume oil, and
water ad 100 %.

The invention relates also to an oral composition containing from 0.01 to 15 % by weight, based on the total weight of the composition, of a compound of formula (1), and orally tolerable adjuvants.

Example of an oral composition:
10 % by weight sorbitol,
10 % by weight glycerol,
15 % by weight ethanol,
15 % by weight propylene glycol,
0.5 % by weight sodium lauryl sulfate,
0.25 % by weight sodium methylcocyl taurate,
0.25 % by weight polyoxypropylene/polyoxyethylene block copolymer,
0.10 % by weight peppermint flavouring,
0.1 to 0.5 % by weight of a compound of formula (1), and
48.6 % by weight water.

The oral composition according to the invention may be, for example, in the form of a gel, a paste, a cream or an aqueous preparation (mouthwash).

The oral composition according to the invention may also comprise compounds that release fluoride ions which are effective against the formation of caries, for example inorganic fluoride salts, e.g. sodium, potassium, ammonium or calcium fluoride, or organic fluoride salts, e.g. amine fluorides, which are known under the trade name Olafluor.

The 4-amino-2-(2-pyridyl)pyrimidines of formula (1) used in accordance with the invention are also suitable for treating, especially preserving, textile fibre materials. Such materials are undyed and dyed or printed fibre materials, e.g. of silk, wool, polyamide or polyurethanes, and especially cellulosic fibre materials of all kinds. Such fibre materials are, for example, natural cellulose fibres, such as cotton, linen, jute and hemp, as well as cellulose and regenerated cellulose. Preferred suitable textile fibre materials are made of cotton.

The 4-amino-2-(2-pyridyl)pyrimidines according to the invention are suitable also for treating, especially imparting antimicrobial properties to or preserving, plastics, e.g. polyethylene, polypropylene, polyurethane, polyester, polyamide, polycarbonate, latex etc.. Fields of use therefor are, for example, floor coverings, plastics coatings, plastics containers and packaging materials; kitchen and bathroom utensils (e.g. brushes, shower curtains, sponges, bathmats), latex, filter materials (air and water filters), plastics articles used in the field of medicine, e.g. dressing materials, syringes, catheters etc., so-called "medical devices", gloves and mattresses.

Paper, for example papers used for hygiene purposes, may also be provided with antimicrobial properties using the 4-amino-2-(2-pyridyl)pyrimidines according to the invention.

It is also possible for nonwovens, e.g. nappies/diapers, sanitary towels, panty liners, and cloths for hygiene and household uses, to be provided with antimicrobial properties in accordance with the invention.

The 4-amino-2-(2-pyridyl)pyrimidines of formula (1) are also used in washing and cleaning formulations, e.g. in liquid or powder washing agents or softeners.

The 4-amino-2-(2-pyridyl)pyrimidines of formula (1) can also be used especially in household and general-purpose cleaners for cleaning and disinfecting hard surfaces.

A cleaning preparation has, for example the following composition:
0.01 to 5 % by weight of a compound of formula (1)
3.0 % by weight octyl alcohol 4EO
1.3 % by weight fatty alcohol C₈-C₁₀polyglucoside
3.0 % by weight isopropanol
water ad 100 %.

In addition to preserving cosmetic and household products, the preservation of technical products, the provision of technical products with antimicrobial properties and use as a biocide in technical processes are also possible, for example in paper treatment, especially in paper treatment liquors, printing thickeners of starch or cellulose derivatives, surface-coatings and paints.

The 4-amino-2-(2-pyridyl)pyrimidines of formula (1) are also suitable for the antimicrobial treatment of wood and for the antimicrobial treatment of leather, the preserving of leather and the provision of leather with antimicrobial properties.

The compounds according to the invention are also suitable for the protection of cosmetic products and household products from microbial damage.

The following Examples illustrate, but do not limit, the present invention.

### Preparation Examples

### Example 1: Synthesis of substituted 4-amino-2-(2-pyridyl)pyrimidines

### 1a: Preparation of 2-(2-pyridyl)pyrimidines

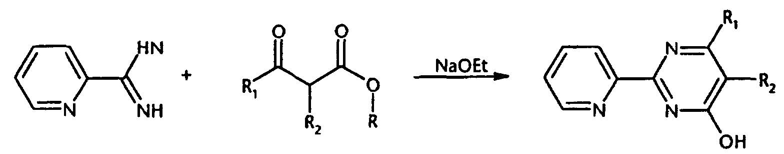

Sodium (1.44 g; 63 mmol) is dissolved, under nitrogen, in absolute ethanol (28.8 ml) at 45°C. A solution of 2-amidinopyridine hydrochloride (9.8 g; 63 mmol) in abs. ethanol (35 ml) is added and the mixture is heated at reflux for 1 hour. Portions, each of 8 ml, of the suspension are transferred to 8 flasks each containing a β-keto ester (7.88 mmol) in abs. ethanol (5 ml) (see Table 2 for amounts used). The suspensions are heated at reflux for 5 hours. After cooling to 25°C, the reaction mixture is evaporated to dryness and directly used in chlorination.

**Tab. 2:**

| β-Keto esters used | | | | |
|---|---|---|---|---|
| β-Keto ester | R₁ | R₂ | R | Amount used |
| 2-ethylacetoacetic acid ethyl ester | -CH₃ | -C₂H₅ | -C₂H₅ | 1.27 g |
| acetoacetic acid ethyl ester | -CH₃ | H | -C₂H₅ | 1.04 g |
| trifluoroacetoacetic acid methyl ester | -CF₃ | H | -CH₃ | 1.36 g |
| 4-methyl-3-oxopentanoic acid methyl ester | -isopropyl | -CF₃ | -CH₃ | 1.27 g |
| 4,4-dimethyl-3-oxopentanoic acid methyl ester | -tert-butyl | H | -CH₃ | 1.27 g |
| 2-isopropylacetoacetic acid ethyl ester | -CH₃ | -isopropyl | -C₂H₅ | 1.38 g |
| 5-methoxy-3-oxopentanoic acid methyl ester | H₃C-O-CH₂-CH₂- | H | -CH₃ | 1.28 g |
| benzoylacetic acid ethyl ester | phenyl | H | -C₂H₅ | 1.54 g |

### b: Synthesis of 4-chloro-2-(2-pyridyl)pyrimidines

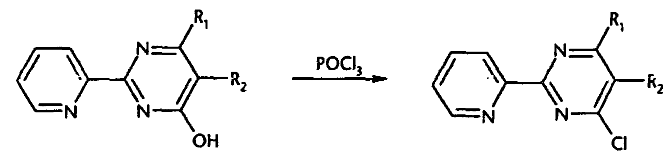

The crude products from a) are taken up in phosphorus oxychloride (5 ml, 54 mmol, in each case) and heated at 110°C for 3 hours. After cooling to 25°C, the reaction mixtures are poured into 10 ml of ice-water and slowly adjusted to pH 8-9 using aqueous sodium hydroxide solution. The crude products obtained are extracted with dichloromethane (5 x 10 ml) and the organic extracts are washed with water (2 x 10 ml) and with saturated NaCl solution. After drying over MgSO₄, the product is filtered off and evaporated to dryness. Because of the good purity of most of the products (see Table 3), further processing is carried out without further purification.

**Tab. 3:**

| HPLC purity of crude products (detection at 214 nm) | | | |
|---|---|---|---|
| No. | R₁ | R₂ | HPLC purity [%] |
| 1 | -CH₃ | -Et | >99 |
| 2 | -CH₃ | -H | >99 |
| 3 | -CF₃ | -H | >99 |
| 4 | -isopropyl | -H | >99 |
| 5 | -tert-butyl | -H | 93 |
| 6 | -CH₃ | -iPr | 83 |
| 7 | -CH₂CH₂-O-CH₃ | -H | 98 |
| 8 | -phenyl | -H | <5 |

### c: Loading of trityl chloride-polystyrene resin (TCP) with diamines

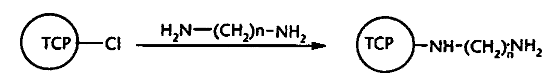

In each case, 1 g of TCP resin (resin loading: 1.44 mmol/g) was shaken in abs. dichloromethane (5 ml) with a diamine (see Tab. 4; 5 equiv.; 7.2 mmol) for 24 hours at 25°C. The resin is washed with dichloromethane (5x), 1 % HOAc/DCM, DMF and diethyl ether and dried *in vacuo*.

**Tab. 4:**

| Diamines used and weights thereof | |
|---|---|
| diamine | amount used |
| n = 7 | 938 mg |
| n = 8 | 1039 mg |
| n = 9 | 1140 mg |
| n = 12 | 1443 mg |

### d: Reaction of the diamine-TCP resins with 4-chloro-2-(2-pyridyl)pyrimidines

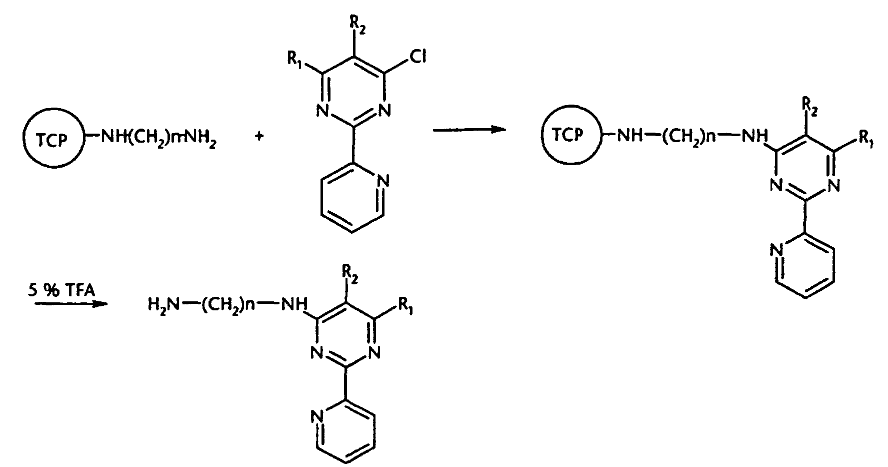

Diamine-TCP resins (50 mg; 72 µmol of diamine, in each case) are shaken in abs. dichloromethane (1 ml, in each case) with 4-chloro-2-(2-pyridyl)pyrimidines (3 equiv.; 216 µmol, in each case) and DIPEA (5 equiv.; 360 µmol) for 48 hours at 25°C. The resin is then filtered off, washed (DMF 5x, MeOH 5x, DCM 5x, diethyl ether 5x) and dried *in vacuo*. Cleaving is then carried out using 5 % TFA/DCM (1.5 mL, in each case) for 1 hour at 25°C. The cleavage solutions are evaporated to dryness and the crude products are freeze-dried from *t*BuOH/water 4:1 with 10 % HOAc and once from *t*BuOH/water 4:1.

### e: Reaction of 4-chloro-2-(2-pyridyl)pyrimidines with monoamines

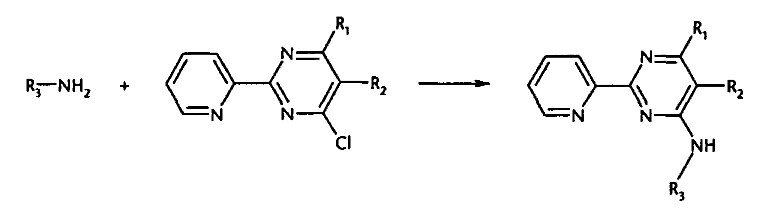

4-Chloro-2-(2-pyridyl)pyrimidines (72 µmol, in each case) are heated with monoamines (3 equiv.; 216 µmol, in each case) in abs. dioxane (0.5 ml) for 24 hours at 90°C. After cooling to 25°C, in order to remove the excess of amine, a scavenger resin (polystyrene aldehyde resin; resin loading 1.28 mmol/g; 3 equiv; 216 mmol; 170 mg) and also trimethyl orthoformate (2 equiv.; 144 µmol; 15 mg; 16 µl) and additional abs. THF (2 ml) are added. The reaction mixture is shaken for 24 hours at 25°C. After filtering, the filtrate is evaporated to dryness and the crude product is freeze-dried from *t*BuOH/water 4:1 with 10 % HOAc and once from *t*BuOH/water 4:1.

**Tab. 5:**

| Amines and amounts used | | | |
|---|---|---|---|
| Amine | Amount (216 µmol) | Amine | Amount (216 µmol) |
| H₂N-(CH₂)₆-CH₃ | 25 mg; 32 µL | H₂N-(CH₂)₁₀-CH₃ | 37 mg; 46 µL |
| H₂N-(CH₂)₇-CH₃ | 28 mg; 36 µL | H₂N-(CH₂)₁₄-CH₃ | 49 mg |
| H₂N-(CH₂)₈-CH₃ | 31 mg; 40 µL | benzylamine | 23 mg; 24 µL |
| H₂N-(CH₂)₉-CH₃ | 34 mg; 43 µL | cyclohexylamine | 21 mg; 25 µL |
| H₂N-(CH₂)₁₁-CH₃ | 40 mg; 50 µL | phenylethylamine | 26 mg; 29 µL |
| H₂N-(CH₂)₁₅-CH₃ | 52 mg | | |

All compounds prepared by the methods described above are listed in Tab. 1 and were characterised by means of HPLC and MS (Table 7, Purities). Some of the compounds were analysed using ¹H-NMR spectroscopy (Table 6):

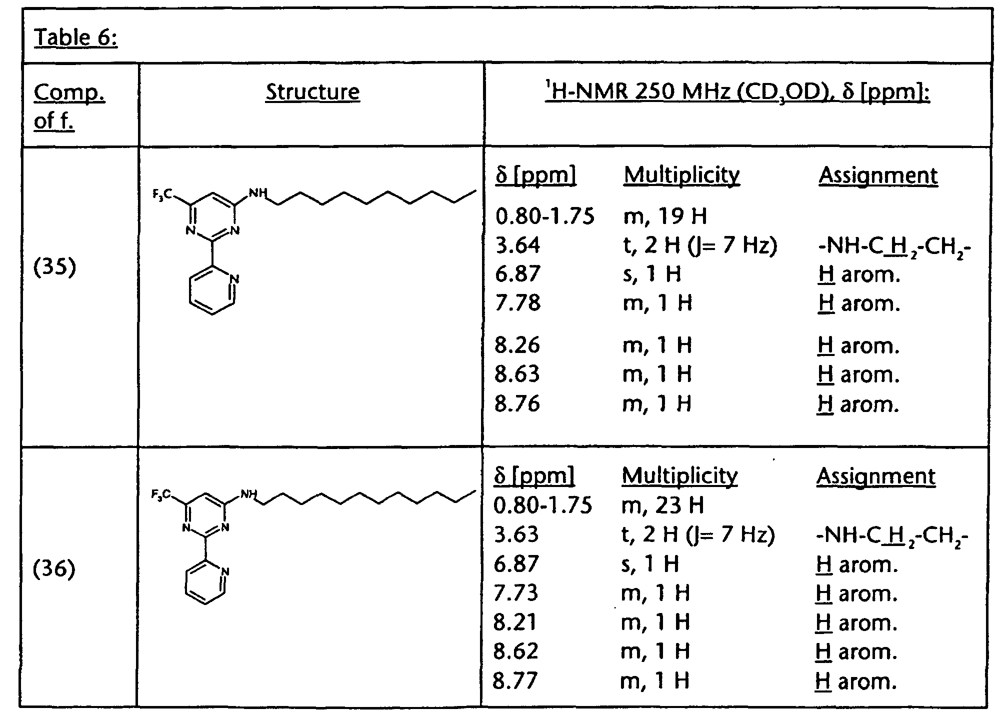

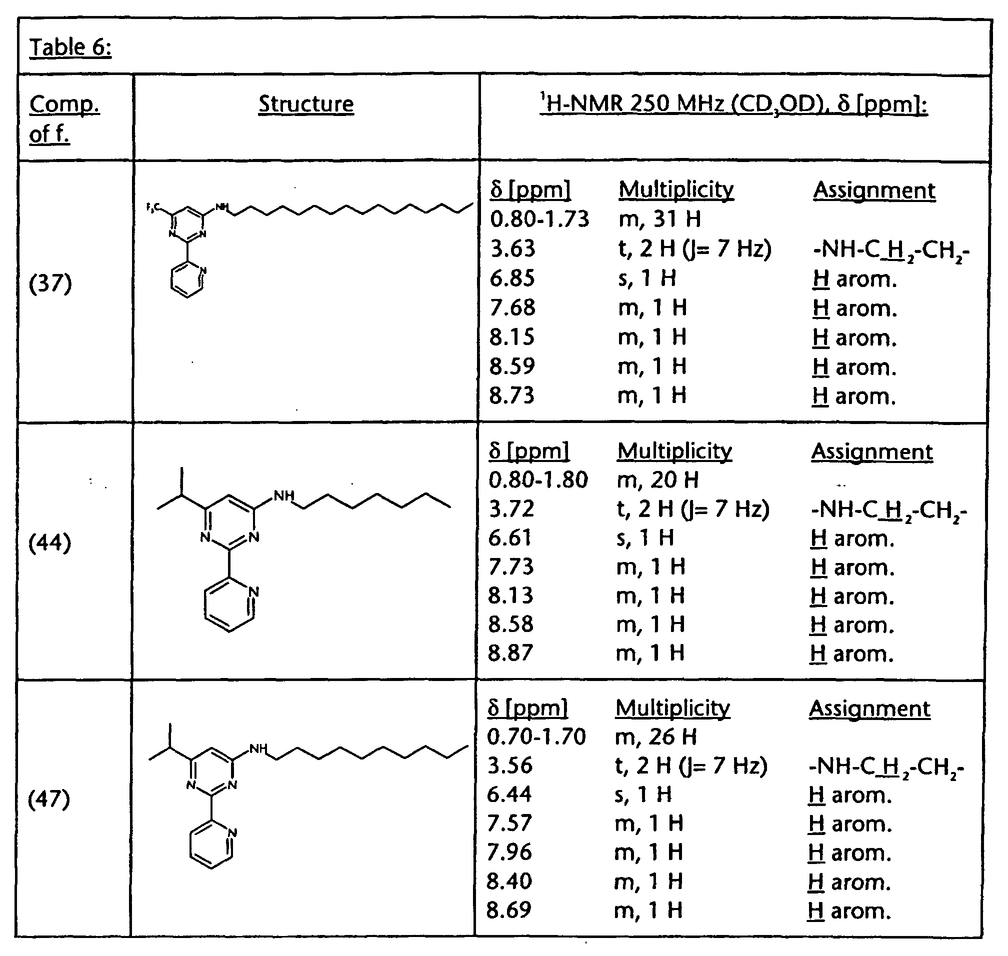

### Example 2: Determination of the minimum inhibitory concentration (MIC value) in microtitre plates

### Nutrient medium:

Casein-soybean flour-peptone broth for preparation of pre-cultures of test bacteria and yeast.
Mycological slant agar for the pre-culture of moulds

### Examples of test organisms:

- Bacteria:: Staphylococcus hominis DMS 20328 (= SH)
Escherichia coli NCTC 8196 (= EC)

### Procedure:

The test substances are pre-dissolved in dimethyl sulfoxide (DMSO) and tested in a dilution series of 1:2.

Bacteria and yeast are cultured overnight in CASO broth, the mould is cultured overnight on mycological slant agar, and washed off using 10 ml of 0.85 % sodium chloride solution (+0.1 % Triton X-100).

All the test organisms are adjusted to an organism count of 1- 5 x 10⁶ CFU/ml using 0.85 % sodium chloride solution.

The test substances are pre-pipetted into microtitre plates in amounts of 8 µl per well.

Pre-diluted organism suspensions are diluted 1:100 in CASO broth (bacteria and yeast) or Sabouraud 2% glucose broth (mould) and are added in amounts of 192 µl per well to the test substances.

The test batches are incubated for 48 hours at 37°C (bacteria and yeast) or for 5 days at 28°C (mould).

After incubation, the growth is determined on the basis of the turbidity of the test batches (optical density) at 620 nm in a microplate reader.

The minimum inhibitory concentration (MIC value) is the concentration of substance at which there is found (compared to the growth of the control) an appreciable inhibition of growth (≤ 20 % growth) of the test organisms.

One microtitre plate is used for each test organism and substance concentration. All the substances are tested in duplicate.

The results are compiled in Table 7:

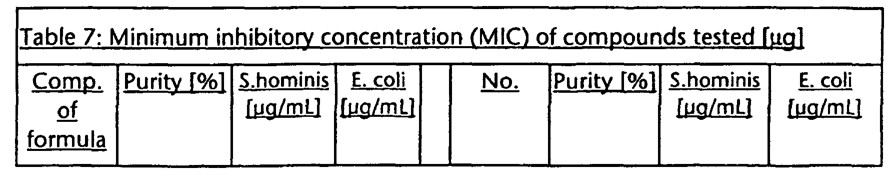

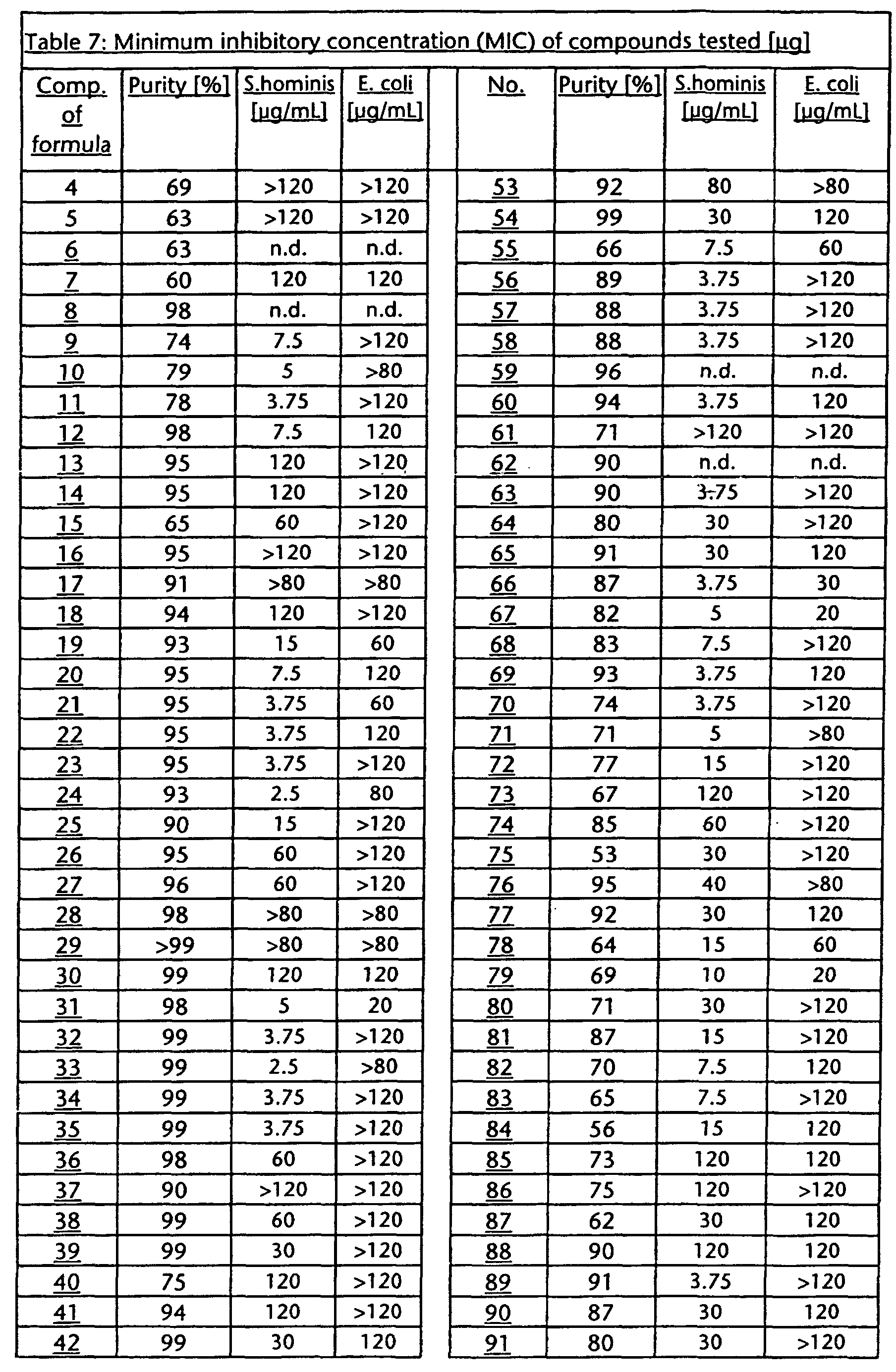

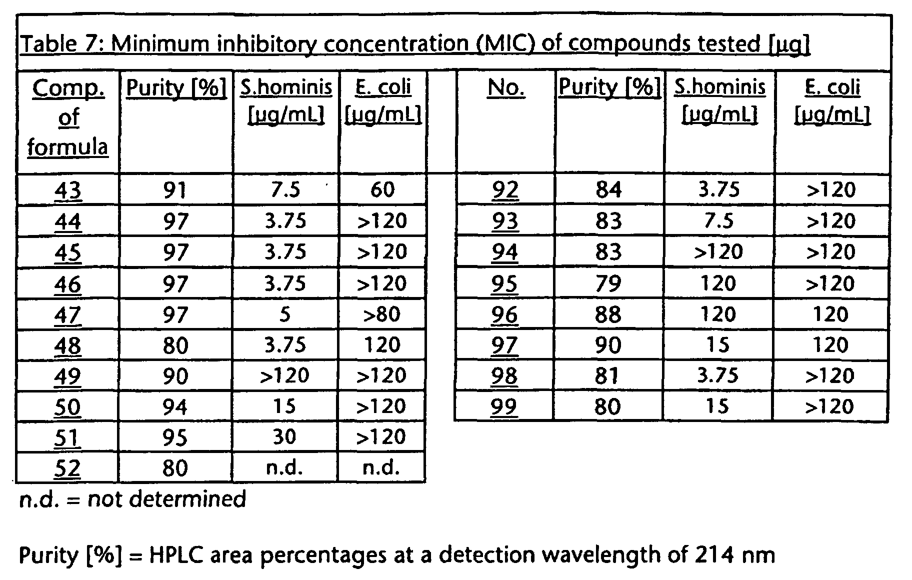

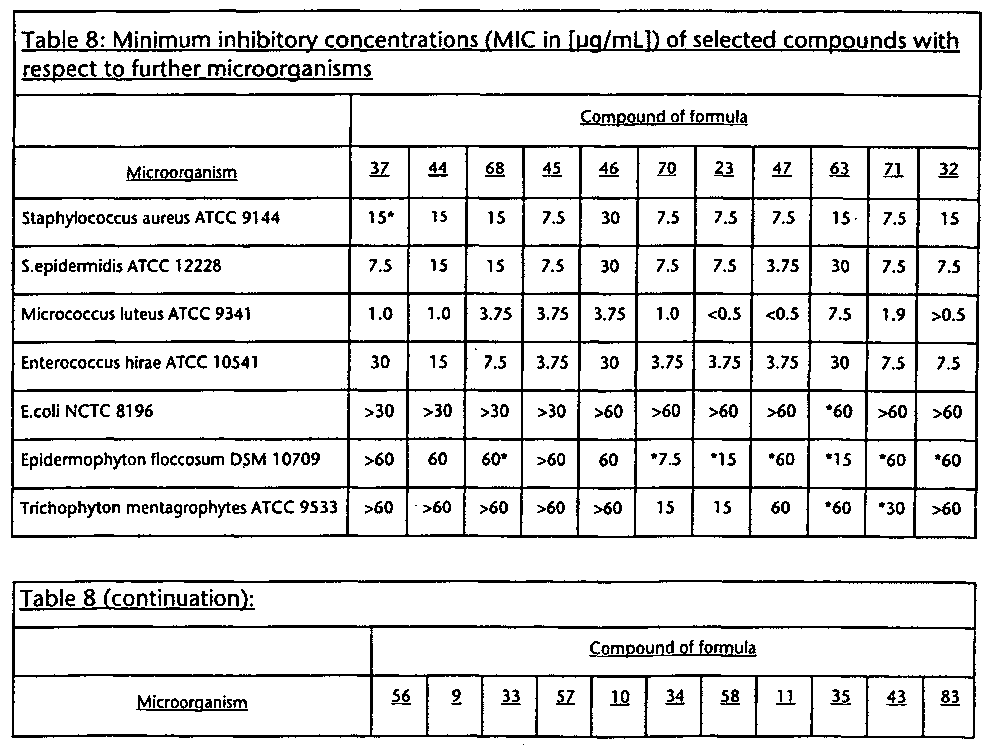

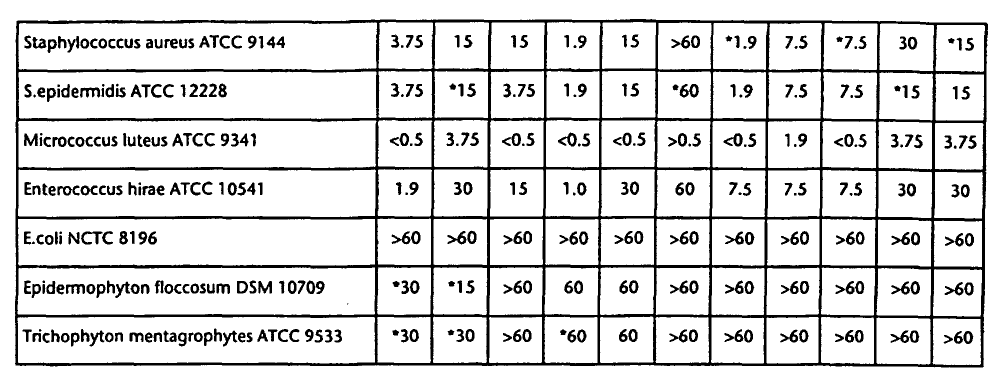

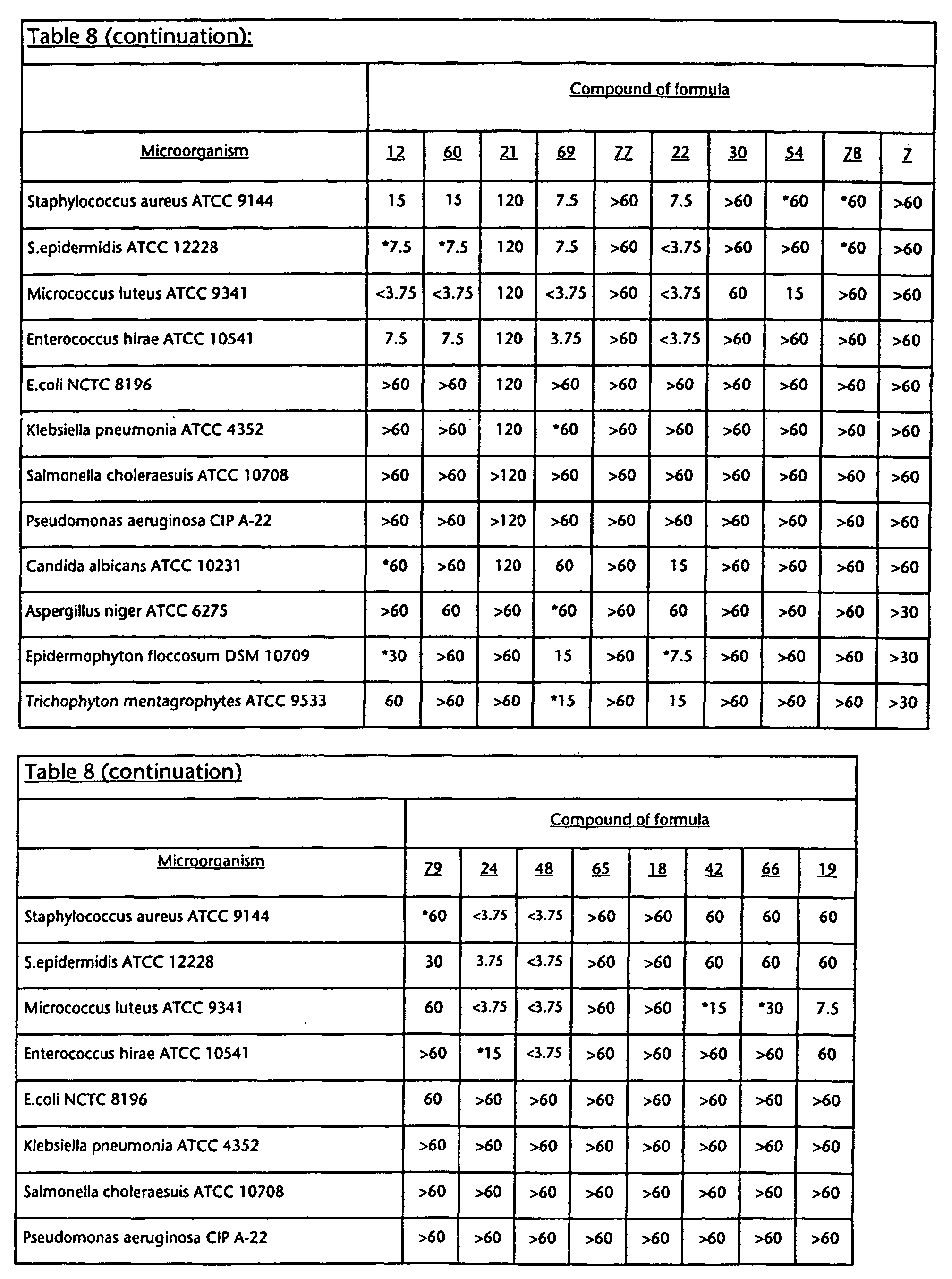

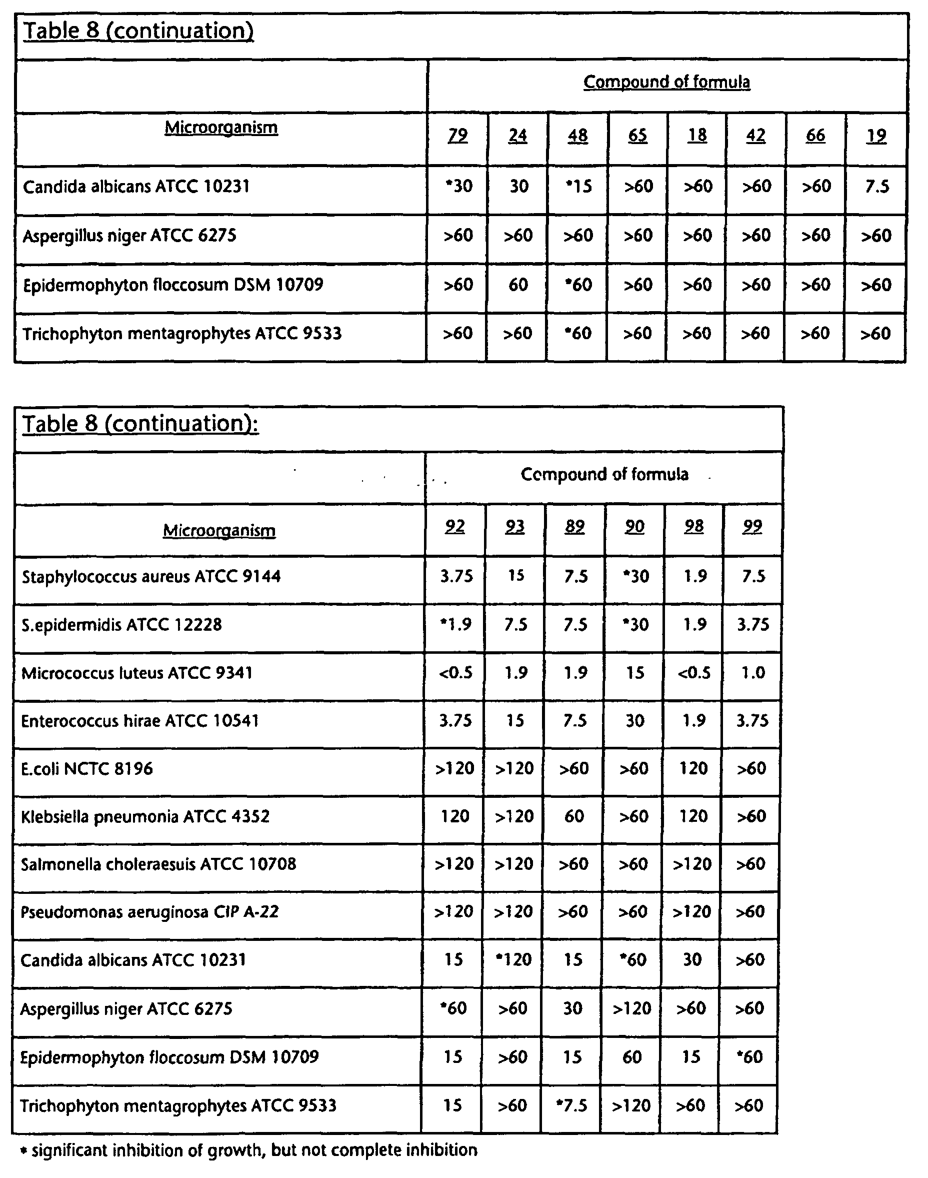

### Example 3: Determination of the bactericidal activity of selected compounds

### Test method:

Nutrient medium:
Casein-soybean flour-peptone broth for preparation of pre-cultures of test bacteria

Examples of test organisms:
Staphylococcus aureus ATCC 6538
Escherichia coli ATCC 10536
Salmonella choleraesuis ATCC 10708

### Procedure:

The test substances are dissolved in dimethyl sulfoxide (DMSO) and tested in a concentration of 120 µg/ml.

Bacteria are incubated overnight in CASO broth and adjusted to an organism count of 1 - 5 x 10⁵ CFU/ml using 0.85 % sodium chloride solution.

The test substances are pre-pipetted into microtitre plates in amounts of 8 µl per well.

The adjusted test organism suspensions are added in amounts of 192 µl per well to the test substances and mixed. After defined contact times, the test batches are mixed, an aliquot is withdrawn and diluted in several steps in a dilution series of 1:10 in a suitable inactivation medium.

The test plates are incubated for 24 hours at 37°C.

After incubation, the growth is determined on the basis of the turbidity of the test batches (optical density) at 620 nm in a microplate reader.

On the basis of the number of growth-exhibiting steps in the dilution series, the reduction in the test organism concentration is determined in powers of ten (log value).

One microtitre plate is used for each test organism.

All the substances are tested in duplicate.

**Table 9**

| Logarithmic reduction in organism count after contact for 30 minutes at a substance concentration of 120 µg/mL | | | |
|---|---|---|---|
| | | Microorganism | |
| Compound of formula | S. choleraesuis ATCC 10708 | E. coli NCTC 8196 | S. aureus ATCC 6538 |
| (12) | <1 | <1 | 1-2 |
| (37) | <1 | <1 | 1-2 |
| (44) | <1 | <1 | 1-2 |
| (60) | <1 | <1 | <1 |
| (68) | <1 | <1 | ≤1 |
| (21) | <1 | <1 | 1-2 |
| (45) | <1 | <1 | 1-2 |
| (69) | ≤1 | <1 | 1-2 |
| (77) | <1 | <1 | >3 |
| (22) | <1 | <1 | 1-2 |
| (30) | ≤1 | <1 | ≤1 |
| (46) | <1 | <1 | ≤1 |
| (54) | ≤1 | ≤1 | 2-3 |
| (70) | <1 | ≤1 | ≤1 |
| (78) | <1 | ≤1 | >3 |
| (7) | <1 | ≤1 | 2 |
| (23) | <1 | <1 | 3 |
| (47) | <1 | <1 | 1 |
| (63) | <1 | ≤1 | <1 |
| (71) | <1 | <1 | 2 |
| (24) | <1 | ≤1 | 2 |
| (32) | ≤1 | <1 | <1 |
| (48) | <1 | <1 | 1 |
| (57) | <1 | <1 | 1 |
| (9) | <1 | <1 | <1 |
| (33) | <1 | <1 | <1 |
| (57) | <1 | <1 | <1 |
| (65) | <1 | <1 | 3 |
| (10) | <1 | <1 | 1-2 |
| (18) | <1 | <1 | 1 |
| (34) | <1 | <1 | <1 |
| (42) | <1 | ≤1 | 2 |
| (58) | ≤1 | ≤1 | 1 |
| (11) | <1 | ≤1 | 1-2 |
| (19) | <1 | ≤1 | 2-3 |
| (35) | <1 | <1 | <1 |
| (43) | <1 | <1 | 3 |
| (83) | <1 | ≤1 | 2 |
| (92) | ≤1 | <1 | >3 |
| (93) | <1 | ≤1 | 2 |
| (89) | ≤1 | ≤1 | 2 |
| (90) | <1 | <1 | 2 |
| (98) | 2-3 | 1.2 | 1 |
| (99) | <1 | ≤1 | 2 |

## Claims

1. A compound of formula 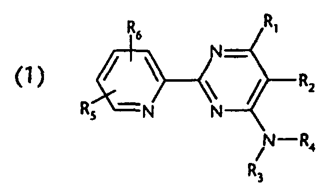 wherein
R₁ and R₂ are each independently of the other hydrogen; unsubstituted or mono- or poly-halo-substituted C₁-C₂₀alkyl, C₁-C₂₀alkoxy, C₂-C₂₀alkenyl, C₂-C₂₀alkynyl, C₃-C₁₈cycloalkyl, C₃-C₇cycloalkyl-C₁-C₂₀alkyl; hydroxy; C₁-C₆alkoxy-C₁-C₂₀alkyl; carboxy; C₁-C₆alkyloxycarbonyl; cyano; mono- or di-C₁-C₂₀alkylamino; C₁-C₆alkylamino-C₁-C₂₀alkyl; halogen; phenyl; unsubstituted or C₁-C₅alkyl-, halo- or hydroxy-substituted phenyl-C₁-C₂₀alkyl, phenoxy or phenyl-C₁-C₂₀alkoxy; or R₁ and R₂ form a polymethylene chain of formula -(CH₂)ₘ- wherein m = 2-12;
R₃ is unsubstituted C₇-C₂₀alkyl; or amino-, hydroxy-, carboxy- or C₁-C₆alkyloxycarbonyl-substituted C₂-C₂₀alkyl, C₈-C₁₈cycloalkyl, C₈-C₂₀alkenyl, C₈-C₂₀alkynyl, C₃-C₇cycloalkyl-C₈-C₂₀alkyl, C₁-C₄alkoxy-C₈-C₂₀alkyl, R₇R₈N-C₇-C₂₀alkyl, phenyl, phenyl-C₁-C₄alkyl or phenyl-C₁-C₄alkoxy;
R₄ is hydrogen; unsubstituted or C₁-C₅alkyl-, halo- or hydroxy-substituted C₁-C₂₀alkyl, C₂-C₂₀alkenyl, C₂-C₂₀alkynyl, C₃-C₂₀cycloalkyl, C₃-C₇cycloalkyl-C₁-C₂₀alkyl, C₁-C₂₀alkoxy-C₁-C₆alkyl or R₇R₈N-C₁-C₂₀alkyl, phenyl, phenyl-C₁-C₂₀alkyl or phenoxy-C₁-C₂₀alkyl;
R₅ and R₆ are each independently of the other hydrogen; and
**R**_{**7**} **and R**_{**8**} **are each independently of the other hydrogen; C**_{**1**}**-C**_{**20**}**alkyl; C**_{**3**}**-C**_{**20**}**alkenyl; C**_{**3**}**- C**_{**20**}**alkynyl; C**_{**3**}**-C**_{**7**}**cycloalkyl; C**_{**3**}**-C**_{**20**}**cycloalkyl-C**_{**1**}**-C**_{**4**}**alkyl; phenyl; or phenyl-C**_{**1**}**-C**_{**4**}**alkyl.**

2. A compound according to claim 1, wherein
R₁ and R₂ are each independently of the other hydrogen, unsubstituted or mono- or poly-halo-substituted C₁-C₂₀alkyl; or C₁-C₅alkoxy-C₁-C₅alkyl.

3. A compound according to claim 2, wherein
R₁ and R₂ are each independently of the other hydrogen, unsubstituted or mono- or poly-halo-substituted C₁-C₅alkyl; or C₁-C₃alkoxy-C₁-C₅alkyl.

4. A compound according to claim 2 or claim 3, wherein
R₁ and R₂ are each independently of the other hydrogen, methyl, ethyl, isopropyl, tert-butyl, CF₃ or the radical -(CH₂)ₘ-O-CH₃ and
m is from 1 to 4.

5. A compound according to any one of claims 1 to 4, wherein
R₃ is unsubstituted or amino-substituted C₇-C₂₀alkyl, C₃-C₇cycloalkyl-C₈-C₂₀alkyl, C₁-C₄-alkoxy-C₇-C₂₀alkyl, R₇R₈N-C₇-C₂₀alkyl, phenyl-C₁-C₄alkyl or phenyl-C₁-C₆alkoxy; and
R₁, R₂, R₄, R₅, R₆, R₇ and R₈ are as defined in claim 1.

6. A compound according to claim 5, wherein
R₃ is unsubstituted or amino-substituted C₇-C₂₀alkyl or R₇R₈N-C₇-C₂₀alkyl.

7. A compound according to claim 5, wherein
R₃ is phenyl-C₁-C₃alkyl.

8. A compound according to claim 1, wherein
R₁ and R₂ are each independently of the other hydrogen, methyl, ethyl, isopropyl, tert-butyl, CF₃ or the radical -(CH₂)ₘ-O-CH₃;
R₃ is unsubstituted or amino-substituted C₇-C₂₀alkyl or R₇R₈N-C₇-C₂₀alkyl;
R₄ is hydrogen; or C₁-C₂₀alkyl;
R₅ **and** R₆ are hydrogen;
**R**_{**7**} **and R**_{**8**} **are each independently of the others hydrogen or C1-C**_{**20**}**alkyl;** and
m is from 1 to 4.

9. A compound according to claim 1, which corresponds to formula 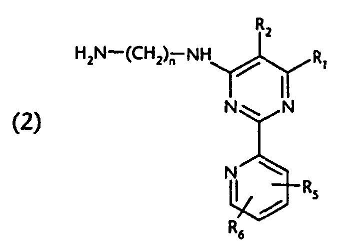 wherein
n is from 7 to 20; and
R₁, R₂, R₅ and R₆ are as defined in claim 1.

10. A compound according to claim 1, which corresponds to formula 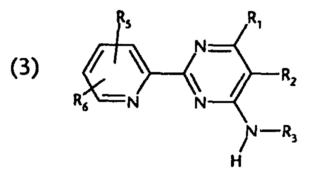 wherein
R₁, R₂, R₃, R₅ and R₆ are as defined in claim 1.

11. A process for the preparation of a compound of formula 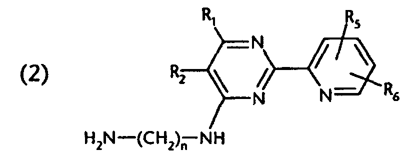 according to claim 1, which comprises preparing the compound in a solid-phase synthesis using a trityl resin (TCP) according to the following scheme: 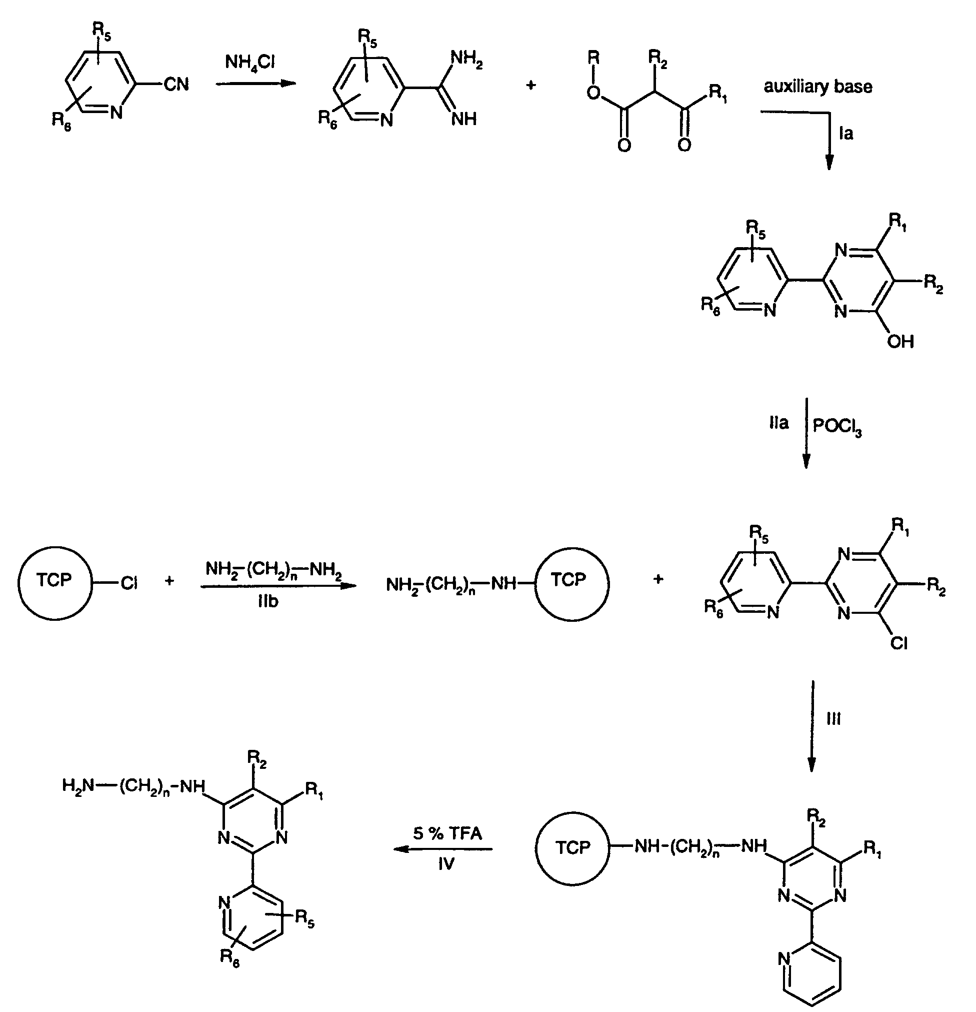 wherein
R = alkyl and
R₁, R₂, R₅, R₆ and n are as defined in claim 1.

12. A process for the preparation of a compound of formula 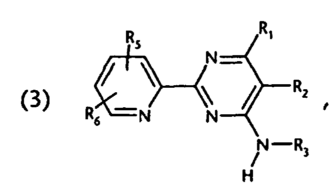 which comprises reacting 2-amidinopyridine with a keto ester using an auxiliary base in a suitable solvent according to the following scheme: 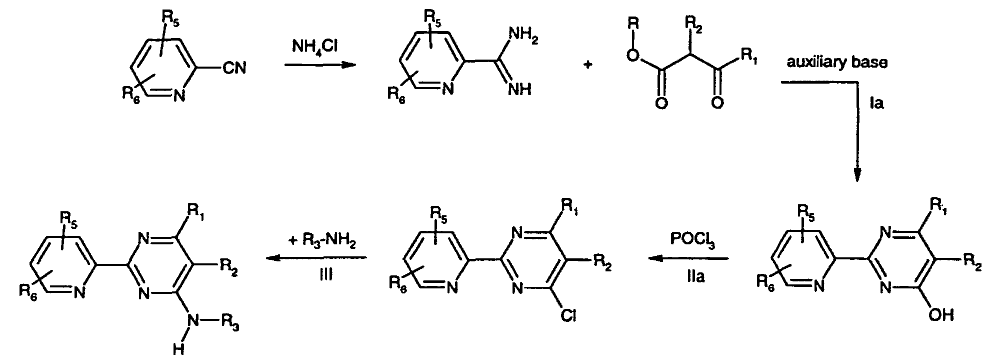 wherein
R₁, R₂, R₃, R₅ and R₆ are as defined in claim 1.

13. Use of a compound of formula (1) according to claim 1 for the antimicrobial treatment of surfaces.

14. Use of a compound of formula (1) for the antimicrobial treatment, deodorisation and disinfection of the skin, mucosa and hair.

15. Use according to claim 13, wherein the compound of formula (1) is used for disinfection and deodorisation.

16. Use of a compound of formula (1) for the treatment of textile fibre materials.

17. Use according to claim 13, wherein the compound of formula (1) is used for preservation.

18. Use of a compound of formula (1) in washing and cleaning formulations.

19. Use of a compound of formula (1) in imparting antimicrobial properties to, and preserving, plastics, paper, nonwovens, wood or leather.

20. Use of a compound of formula (1) in imparting antimicrobial properties to, and preserving, technical products, especially printing thickeners of starch or of cellulose derivatives, surface-coatings and paints.

21. Use of a compound of formula (1) as a biocide in technical processes.

22. A personal care preparation comprising
from 0.01 to 15 % by weight, based on the total weight of the composition, of a compound of formula (1), and cosmetically tolerable adjuvants.

23. An oral composition comprising from 0.01 to 15 % by weight, based on the total weight of the composition, of a compound of formula (1), and orally tolerable adjuvants.

## Patentansprüche

1. Verbindungen der Formel 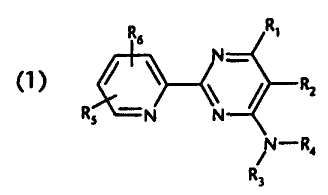 worin
R₁ und R₂ unabhängig voneinander Wasserstoff; nicht substituiertes oder mit einem oder mehreren Halogenatomen substituiertes C₁-C₂₀-Alkyl, C₁-C₂₀-Alkoxy, C₂-C₂₀-Alkenyl, C₂-C₂₀-Alkinyl, C₃-C₁₈-Cycloalkyl, C₃-C₇-Cycloalkyl-C₁-C₂₀-Alkyl; Hydroxy; C₁-C₆-Alkoxy-C₁-C₂₀-Alkyl; Carboxy; C₁-C₆-Alkyl-oxycarbonyl; Cyano; Mono- oder Di-C₁-C₂₀-Alkylamino; C₁-C₆-Alkylamino-C₁-C₂₀-Alkyl; Halogen; Phenyl; nicht substituiertes oder mit C₁-C₅-Alkyl, Halogen oder Hydroxy substituiertes Phenyl-C₁-C₂₀-Alkyl, Phenoxy oder Phenyl-C₁-C₂₀-Alkoxy; oder R₁ und R₂ eine Polymethylenkette der Formel -(CH₂)ₘ- mit m = 2-12 bilden;
R₃ nicht substituiertes C₇-C₂₀-Alkyl; oder mit Amino, Hydroxy, Carboxy oder C₁-C₆-Alkyloxycarbonyl substituiertes C₂-C₂₀-Alkyl, C₈-C₁₈-Cycloalkyl, C₈-C₂₀-Alkenyl, C₈-C₂₀-Alkinyl, C₃-C₇-Cycloalkyl-C₈-C₂₀-Alkyl, C₁-C₄-Alkoxy-C₈-C₂₀-Alkyl, R₇R₈N-C₇-C₂₀-Alkyl, Phenyl, Phenyl-C₁-C₄-Alkyl oder Phenyl-C₁-C₄-Alkoxy;
R₄ Wasserstoff; nicht substituiertes oder mit C₁-C₅-Alkyl, Halogen oder Hydroxy substituiertes C₁-C₂₀-Alkyl, C₂-C₂₀-Alkenyl, C₂-C₂₀-Alkinyl, C₃-C₂₀-Cycloalkyl, C₃-C₇-Cycloalkyl-C₁-C₂₀-Alkyl C₁-C₂₀-Alkoxy-C₁-C₆-Alkyl oder R₇R₈N-C₁-C₂₀-Alkyl, Phenyl, Phenyl-C₁-C₂₀-Alkyl oder Phenoxy-C₁-C₂₀-Alkyl;
R₅ und R₆ unabhängig voneinander Wasserstoff; und
R₇ und R₈ unabhängig voneinander Wasserstoff; C₁-C₂₀-Alkyl; C₃-C₂₀-Alkenyl; C₃-C₂₀-Alkinyl; C₃-C₇-Cycloalkyl; C₃-C₂₀-Cycloalkyl-C₁-C₄-Alkyl;, Phenyl; Phenyl-C₁-C₄-Alkyl; bedeuten.

2. Verbindungen nach Anspruch 1, **dadurch gekennzeichnet, dass**
R₁ und R₂ unabhängig voneinander Wasserstoff, nicht substituiertes oder mit einem oder mehreren Halogenatomen substituiertes C₁-C₂₀-Alkyl; oder C₁-C₅-Alkoxy-C₁-C₅-Alkyl bedeuten.

3. Verbindungen nach Anspruch 2, **dadurch gekennzeichnet, dass** R₁ und R₂ unabhängig voneinander Wasserstoff; nicht substituiertes oder mit einem oder mehreren Halogenatomen substituiertes C₁-C₅-Alkyl; oder C₁-C₃-Alkoxy-C₁-C₅-Alkyl bedeuten.

4. Verbindungen nach Anspruch 2 oder 3, **dadurch gekennzeichnet, dass**
R₁ und R₂ unabhängig voneinander Wasserstoff, Methyl, Ethyl, iso-Propyl, t. Butyl, CF₃ oder den Rest -(CH₂)ₘ-O-CH₃ und
m 1 bis 4
bedeuten.

5. Verbindungen nach einem der Ansprüche 1 bis 4, **dadurch gekennzeichnet, dass**
R₃ nicht substituiertes oder mit Amino substituiertes C₇-C₂₀-Alkyl, C₃-C₇-Cycloalkyl-C₈-C₂₀-Alkyl, C₁-C₄-Alkoxy-C₇-C₂₀-Alkyl, R₇R₈N-C₇-C₂₀-Alkyl, Phenyl-C₁-C₄-Alkyl oder Phenyl-C₁-C₄-Alkoxy; bedeutet und
R₁, R₂, R₄, R₅, R₆, R₇ und R₈ die in Anspruch 1 angegebene Bedeutung haben.

6. Verbindungen nach Anspruch 5, **dadurch gekennzeichnet, dass**
R₃ nicht substituiertes oder mit Amino substituiertes C₇-C₂₀-Alkyl oder R₇R₈N-C₇-C₂₀-Alkyl bedeutet.

7. Verbindungen nach Anspruch 5, **dadurch gekennzeichnet, dass**
R₃ Phenyl-C₁-C₃-Alkyl
bedeutet.

8. Verbindungen nach Anspruch 1, **dadurch gekennzeichnet, dass**
R₁ und R₂ unabhängig voneinander Wasserstoff, Methyl, Ethyl, iso-Propyl, t. Butyl, CF₃ oder den Rest -(CH₂)ₘ-O-CH₃;
R₃ nicht substituiertes oder mit Amino substituiertes C₇-C₂₀-Alkyl oder R₇R₈N-C₇-C₂₀-Alkyl;
R₄ Wasserstoff; oder C₁-C₂₀-Alkyl;
R₅ und R₆ Wasserstoff;
R₇ und R₈ unabhängig voneinander Wasserstoff oder C₁-C₂₀-Alkyl; und
m 1 bis 4
bedeuten.

9. Verbindungen nach Anspruch 1, **dadurch gekennzeichnet, dass** sie der Formel 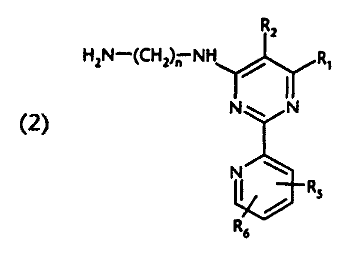 entsprechen, worin
n 7 bis 20; bedeutet und
R₁, R₂, R₅, R₆ die in Anspruch 1 angegebene Bedeutung haben.

10. Verbindungen nach Anspruch 1, **dadurch gekennzeichnet, dass** sie der Formel 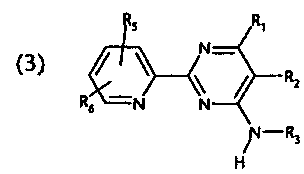 entsprechen, worin
R₁, R₂, R₃, R₅ und R₆ die in Anspruch 1 angegebene Bedeutung haben.

11. Verfahren zur Herstellung der Verbindungen der Formel 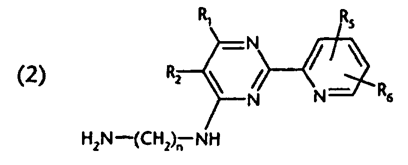 nach Anspruch 1, **dadurch gekennzeichnet, dass** sie in
einer Festphasensynthese unter Verwendung eines Tritylharzes (TCP) nach folgendem Schema hergestellt werden: 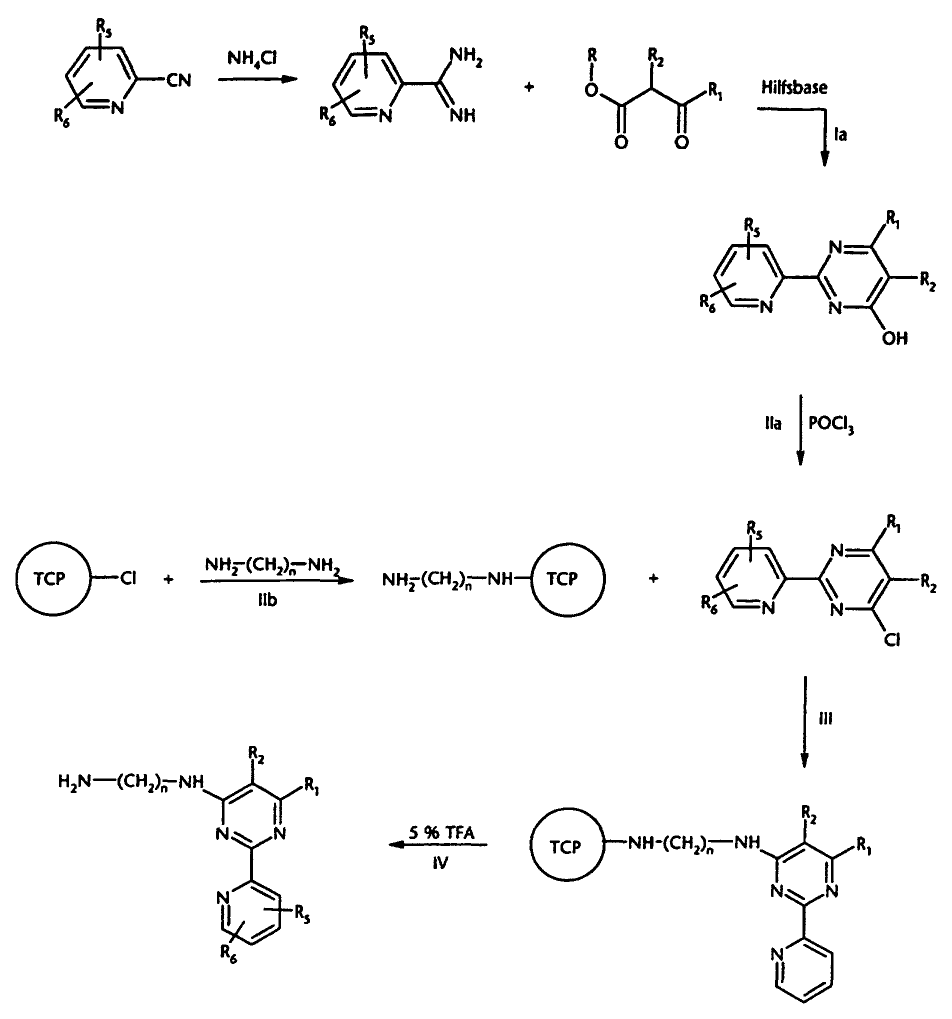 worin
R = Alkyl und
R₁, R₂, R₅, R₆ und n die in Anspruch 1 angegebene Bedeutung haben.

12. Verfahren zur Herstellung der Verbindungen der Formel 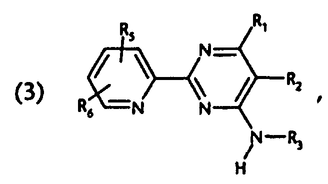 **dadurch gekennzeichnet, dass** 2-amidinopyridin mit einem Ketoester unter Verwendung einer Hilfsbase in einem geeigneten Lösungsmittel nach folgendem Schema umgesetzt wird: 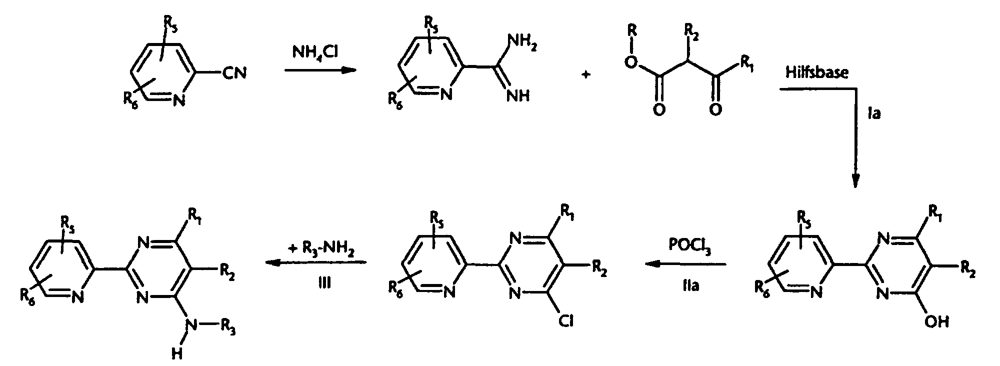 worin
R₁, R₂, R₃, R₅ und R₆ die in Anspruch 1 angegebene Bedeutung haben.

13. Verwendung der Verbindungen der Formel (1) nach Anspruch 1 zur antimikrobiellen Behandlung von Oberflächen.

14. Verwendung der Verbindung der Formel (1) zur antimikrobiellen Behandlung, Desodorierung und Desinfektion der Haut, Schleimhäute und Haare.

15. Verwendung nach Anspruch 13, **dadurch gekennzeichnet, dass** die Verbindung der Formel (1) zur Desinfektion und Desodorierung verwendet wird.

16. Verwendung der Verbindung der Formel (1) zur Behandlung von textilen Fasermaterialien.

17. Verwendung nach Anspruch 13, **dadurch gekennzeichnet, dass** die Verbindung der Formel (1) zur Konservierung verwendet wird.

18. Verwendung der Verbindung der Formel (1) in Wasch- und Reinigungsformulierungen.

19. Verwendung der Verbindung der Formel (1) zur antimikrobiellen Ausrüstung und Konservierung von Kunststoffen, Papier, Nonwovens, Holz oder Leder.

20. Verwendung der Verbindung der Formel (1) zur antimikrobiellen Ausrüstung und Konservierung von technischen Produkten, insbesondere Druckverdickern aus Stärke oder Celluloseabkömmlingen, Lacken und Anstrichfarben.

21. Verwendung der Verbindung der Formel (1) als Biozid in technischen Prozessen.

22. Körperpflegemittel, enthaltend
0,01 bis 15 Gew.-%, bezogen auf das Gesamtgewicht der Zusammensetzung, der Verbindung der Formel (1) und kosmetisch verträgliche Hilfsstoffe.

23. Orale Zusammensetzung, enthaltend 0,01 bis 15 Gew.-%, bezogen auf das Gesamtgewicht der Zusammensetzung, der Verbindung der Formel (1) und oral verträgliche Hilfsstoffe.

## Revendications

1. Composé de formule 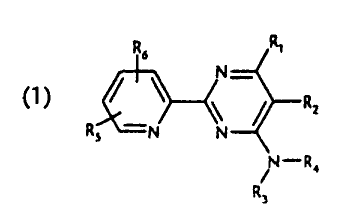 dans laquelle
R₁ et R₂ représentent, chacun indépendamment l'un de l'autre, un atome d'hydrogène ; un groupe alkyle en C₁ à C₂₀ non substitué ou substitué par un groupe mono- ou poly-halogéno, alcoxy en C₁ à C₂₀, alcényle en C₂ à C₂₀, alcynyle en C₂ à C₂₀, cyclolalkyle en C₃ à C₁₈, cycloalkyle en C₃ à C₇-alkyle en C₁ à C₂₀ ; hydroxyle ; alcoxy en C₁ à C₆-alkyle en C₁ à C₂₀ ; carboxyle ; alkyl(en C₁ à C₆)oxycarbonyle ; cyano ; mono- ou di-alkyl(en C₁ à C₂₀)amino ; alkyl(en C₁ à C₆)amino-alkyle en C₁ à C₂₀ ; un atome d'halogène ; un groupe phényle ; phényle-alkyle en C₁ à C₂₀ non substitué ou substitué par un groupe alkyle en C₁ à C₅, halogéno ou hydroxyle, un groupe phénoxy ou phényle-alcoxy en C₁ à C₂₀ ; ou bien R₁ et R₂ forment une chaîne polyméthylène de formule -(CH₂)ₘ- dans laquelle m vaut 2 à 12 ;
R₃ représente un groupe alkyle en C₇ à C₂₀ non substitué ; ou alkyle en C₂ à C₂₀ substitué par un groupe amino, hydroxyle, carboxyle ou alkyl (en C₂ à C₆)oxycarbonyle, cycloalkyle en C₈ à C₁₈, alcényle en C₈ à C₂₀, alcynyle en C₈ à C₂₀, cycloalkyle en C₃ à C₇-alkyle en C₈ à C₂₀, alcoxy en C₁ à C₄-alkyle en C₈ à C₂₀, R₇R₈N-alkyle en C₇ à C₂₀, phényle, phényle-alkyle en C₁ à C₄ ou phényle-alcoxy en C₁ à C₄ ;
R₄ représente un atome d'hydrogène ; un groupe alkyle en C₁ à C₂₀ non substitué ou substitué par un groupe alkyle en C₁ à C₅, halogéno ou hydroxyle, alcényle en C₂ à C₂₀, alcynyle en C₂ à C₂₀, cycloalkyle en C₃ à C₂₀, cycloalkyle en C₃ à C₇-alkyle en C₁ à C₂₀, alcoxy en C₁ à C₂₀-alkyle en C₁ à C₆ ou R₇R₈N-alkyle en C₁ à C₂₀, phényle, phényle-alkyle en C₁ à C₂₀ ou phénoxy-alkyle en C₁ à C₂₀ ;
R₅ et R₆ représentent, chacun indépendamment l'un de l'autre, un atome d'hydrogène ; et
R₇ et R₈ représentent, chacun indépendamment l'un de l'autre, un atome d'hydrogène ; un groupe alkyle en C₁ à C₂₀ ; alcényle en C₃ à C₂₀ ; alcynyle en C₃ à C₂₀ ; cycloalkyle en C₃ à C₇ ; cycloalkyle en C₃ à C₂₀-alkyle en C₁ à C₄ ; phényle ; ou phényle-alkyle en C₁ à C₄.

2. Composé selon la revendication 1, dans lequel
R₁ et R₂ représentent, chacun indépendamment l'un de l'autre, un atome d'hydrogène, un groupe alkyle en C₁ à C₂₀ non substitué ou substitué par un groupe mono- ou poly-halogéno ; ou alcoxy en C₁ à C₅-alkyle en C₁ à C₅.

3. Composé selon la revendication 2, dans lequel
R₁ et R₂ représentent, chacun indépendamment l'un de l'autre, un atome d'hydrogène, un groupe alkyle en C₁ à C₅ non substitué ou substitué par un groupe mono- ou poly-halogéno ; ou alcoxy en C₁ à C₃-alkyle en C₁ à C₅.

4. Composé selon la revendication 2 ou 3, dans lequel
R₁ et R₂ représentent, chacun indépendamment l'un de l'autre, un atome d'hydrogène, un groupe méthyle, éthyle, isopropyle, tert-butyle, CF₃ ou le radical -(CH₂)ₘ-O-CH₃ et
m vaut 1 à 4.

5. Composé selon l'une quelconque des revendications 1 à 4, dans lequel
R₃ représente un groupe alkyle en C₇ à C₂₀ non substitué ou substitué par un groupe amino, cycloalkyle en C₃ à C₇-alkyle en C₈ à C₂₀, alcoxy en C₁ à C₄-alkyle en C₇ à C₂₀, R₇R₈N-alkyle en C₁ à C₂₀, phényle-alkyle en C₁ à C₄ ou phényle-alcoxy en C₁ à C₆ ; et
R₁, R₂, R₄, R₅, R₆, R₇ et R₈ sont tels que définis dans la revendication 1.

6. Composé selon la revendication 5, dans lequel
R₃ représente un groupe alkyle en C₇ à C₂₀ non substitué ou substitué par un groupe amino ou R₇R₈N-alkyle en C₇ à C₂₀ ;

7. Composé selon la revendication 5, dans lequel
R₃ représente un groupe phényle-alkyle en C₁ à C₃.

8. Composé selon la revendication 1, dans lequel
R₁ et R₂ représentent, chacun indépendamment l'un de l'autre, un atome d'hydrogène, un groupé méthyle, éthyle, isopropyle, tert-butyle, CF₃ ou le radical -(CH₂)ₘ-O-CH₃ ;
R₃ représente un groupe alkyle en C₇ à C₂₀ non substitué ou substitué par un groupe amino ou R₇R₈N-alkyle en C₇ à C₂₀ ;
R₄ représente un atome d'hydrogène ou un groupe alkyle en C₁ à C₂₀ ;
R₅ et R₆ représentent un atome d'hydrogène;
R₇ et R₈ représentent, chacun indépendamment l'un de l'autre un atome d'hydrogène ou un groupe alkyle en C₁ à C₂₀; et
m vaut 1 à 4.

9. Composé selon la revendication 1, qui correspond à la formule 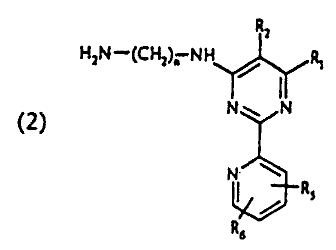 dans laquelle
n vaut 7 à 20 ; et
R₁, R₂, R₅ et R₆ sont tels que définis dans la revendication 1.

10. Composé selon la revendication 1, qui correspond à la formule 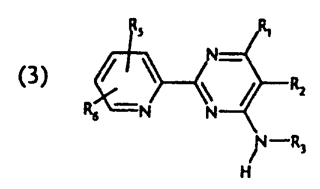 dans laquelle
R₁, R₂, R₃, R₅ et R₆ sont tels que définis dans la revendication 1.

11. Procédé pour la préparation d'un composé de formule 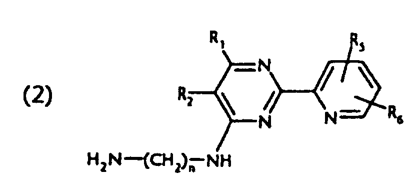 selon la revendication 1, qui consiste à préparer le composé dans une synthèse en phase solide en utilisant une résine trityle (TCP) selon le schéma réactionnel suivant : 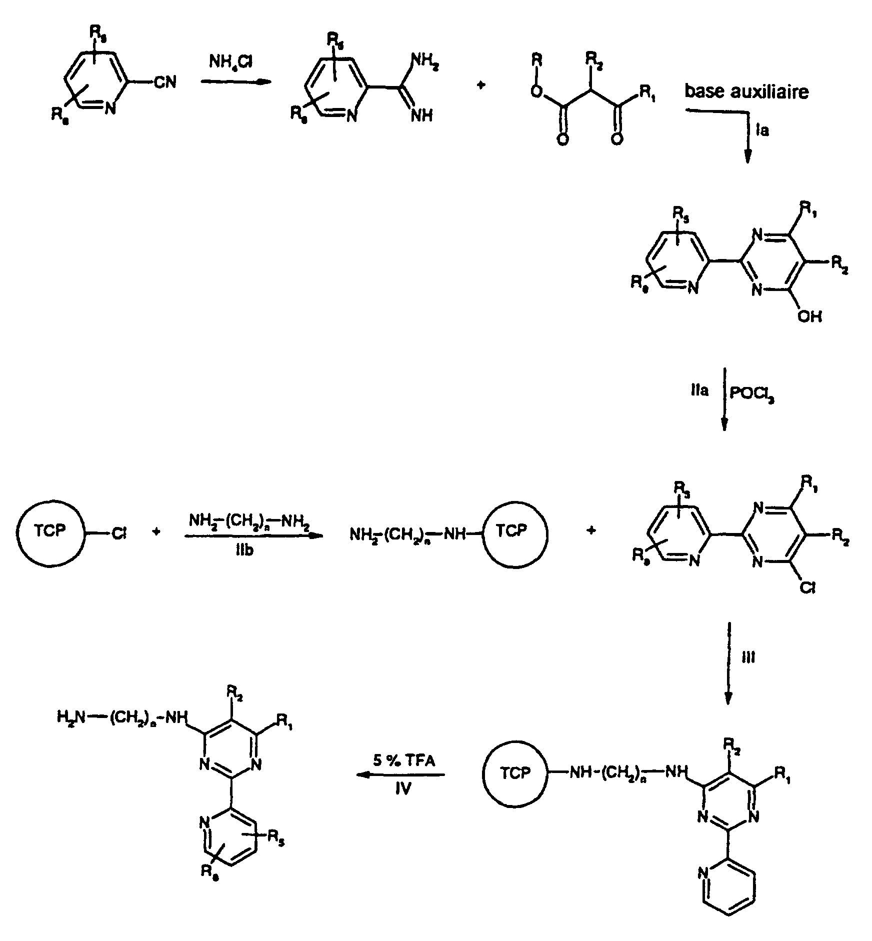 dans lequel
R = alkyle et
R₁, R₂, R₅, R₆ et n sont tels que définis dans la revendication 1.

12. Procédé pour la préparation d'un composé de formule 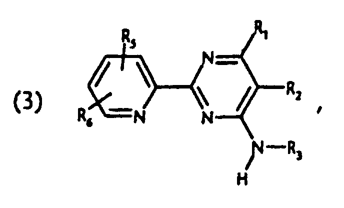 qui consiste à faire réagir la 2-amidinopyridine avec un β-céto-ester en utilisant une base auxiliaire dans un solvant approprié selon le schéma réactionnel suivant : 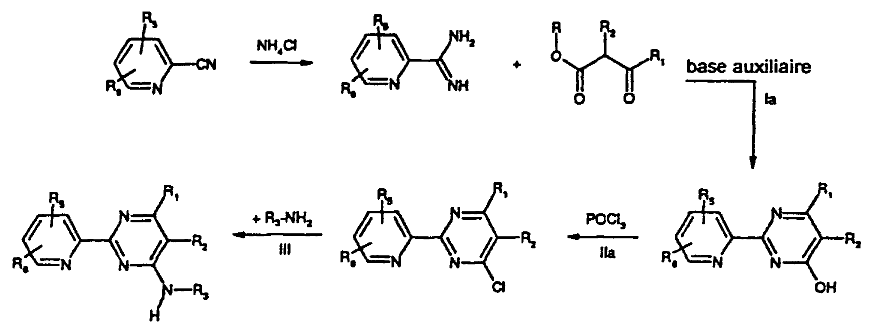 dans lequel
R₁, R₂, R₃, R₅ et R₆ sont tels que définis dans la revendication 1.

13. Utilisation d'un composé de formule (1) selon la revendication 1 pour le traitement antimicrobien de surfaces.

14. Utilisation d'un composé de formule (1) pour le traitement antimicrobien, la désodorisation et la désinfection de la peau, des muqueuses et des cheveux.

15. Utilisation selon la revendication 13, dans laquelle le composé de formule (1) est utilisé pour la désinfection et la désodorisation.

16. Utilisation d'un composé de formule (1) pour le traitement de matières de fibres textiles.

17. Utilisation selon la revendication 13, dans laquelle le composé de formule (1) est utilisé pour la conservation.

18. Utilisation d'un composé de formule (1) dans des formulations lavantes et nettoyantes.

19. Utilisation d'un composé de formule (1) pour conférer des propriétés antimicrobiennes à, et pour conserver, des matières plastiques, du papier, des non-tissés, du bois ou du cuir.

20. Utilisation d'un composé de formule (1) pour conférer des propriétés antimicrobiennes à, et pour conserver, des produits techniques, en particulier des épaississants pour impression à base d'amidon ou de dérivés de cellulose, des revêtements de surface et des peintures.

21. Utilisation d'un composé de formule (1) en tant que biocide dans des procédés techniques.

22. Préparation pour soins personnels comprenant 0,01% à 15% en poids, par rapport au poids total de la composition, d'un composé de formule (1), et des adjuvants acceptables sur le plan cosmétique.

23. Composition orale comprenant 0,01% à 15% en poids, par rapport au poids total de la composition, d'un composé de formule (1), et des adjuvants acceptables par voie orale.
